# FASCICULE DE BREVET EUROPEEN

(11) **EP 0 843 663 B1**
(45) Date de publication et mention de la délivrance du brevet: **09.04.2003**
(21) Numéro de dépôt: 96927111.3
(22) Date de dépôt: 30.07.1996
(51) Int. Cl.: C07D 213/70, C07D 215/36, C07D 213/73, C07D 215/38, C07D 213/74, C07D 401/12, C07D 413/12, C07D 417/12, C07D 213/80, C07D 213/75, C07D 213/64

(54) **4-ARYL-THIO-PYRIDIN-2(1H)-ONES, MEDICAMENTS LES CONTENANT ET LEURS UTILISATIONS DANS LE TRAITEMENT DE MALADIES LIEES AUX VIH**
4-ARYL-THIO-PYRIDIN-2(1H)-ONEN, DIESE ENTHALTENDE ARZNEIMITTEL UND IHRE ANWENDUNGEN BEI DER BEHANDLUNG VON MIT HIV ZUSAMMENHÄNGENDEN KRANKHEITEN
4-ARYL-THIO-PYRIDIN-2(1H)-ONES, DRUGS CONTAINING SAME, AND USES THEREOF FOR TREATING HIV-RELATED DISEASES

(30) Priorité: 31.07.1995 FR 9509323
(43) Date de publication de la demande: 27.05.1998
(73) Titulaire: CENTRE NATIONAL DE LA RECHERCHE SCIENTIFIQUE, 75016 Paris Cédex (FR)
(72) Inventeur: BISAGNI, Emile, F-91400 Orsay (FR); DOLLE, Valérie, F-91400 Orsay (FR); NGUYEN, Chi, Hung, F-91300 Massy (FR); LEGRAVEREND, Michel, F-92160 Antony (FR); AUBERTIN, Anne-Marie, F-67000 Strasbourg (FR); KIRN, André, F-67000 Strasbourg (FR); ANDREOLA, Marie, Line, F-33650 La Brède (FR); TARRAGO-LITVAK, Laura, F-33600 Pessac (FR); VENTURA, Michel, F-33140 Villenave-d'Ornon (FR)
(74) Mandataire: Phélip, Bruno
(86) Numéro de dépôt international: FR9601204
(87) Numéro de publication internationale: WO97005113

(56) Documents cités:
- EP-A- 0 462 800
- J. MED. CHEM., vol. 26, no. 9, 1983, pages 1329-1333, XP000561937 M. CROISY-DELCEY ET AL: cité dans la demande
- ANTI-CANCER DRUG DES., vol. 7, no. 3, 1992, pages 219-233, XP002002674 C. H. NGUYEN ET AL: cité dans la demande
- EUR. J. MED. CHEM. - CHIM. THER., vol. 19, no. 6, 1984, pages 525-528, XP000561943 C. RIVALLE ET AL:
- J. MED. CHEM., vol. 38, no. 23, 10 Novembre 1995, pages 4679-4686, XP002002675 V. DOLL ET AL:

## Description

La présente invention a pour objet des 4-aryl-thio-pyridin-2(1H)-ones et leur application comme médicaments.

Elle est en particulier relative à leurs utilisations dans le traitement des maladies liées aux virus de l'immunodéficience humaine (VIH).

Des dérivés de la 1-[(2-hydroxyéthoxy)méthyl]-6-(phénylthio)thymine (HEPT) et des pyridinones sont connus pour leurs propriétés inhibitrices de la transcriptase inverse du VIH-1.

L'HEPT et un autre dérivé de la même famille, dénommé E-EPU (5-éthyl-1-éthoxyméthyl-6-(phénylthio)uracyle) sont représentés respectivement sur la figure 1 par les formules (1a) et (1b). Des pyridinones présentant des activités inhibitrices vis-à-vis de la transcriptase inverse sont représentées sur la figure 2 par les formules (2a) et (2b).

D'autres composés inhibiteurs de la transcriptase inverse du virus HIV, dont des pyridinones, sont aussi décrits dans la demande de brevet EP-0.462.800 (Merck). Cette demande décrit de manière générale un groupe de pyridinones présentant une structure particulière, dans laquelle le groupe R₄ en position 4 du cycle est un résidu alkylthio ou alkylamino, et le groupe en position 3 est obligatoirement substitué par un résidu aryle ou hétérocyclique éventuellement substitué, lié au noyau pyridinone par une chaîne X-CHnR₃ qui ne peut pas être NHCO. Néanmoins seuls des composés dans lesquels R₄ est un atome d'hydrogène ont été synthétisés. En tout état de cause ce document ne mentionne pas que les composés pénètrent la particule virale.

Ces composés présentent l'inconvénient de provoquer l'apparition rapide de souches résistantes du VIH-1 et leur utilisation au long cours en monothérapie chez l'homme dans le traitement des maladies liées aux virus VIH est donc rendue difficile.

Un autre problème posé dans le traitement des maladies liées aux virus VHI réside dans le blocage de la conversion de l'ARN génomique en ADN proviral, étape indispensable à l'intégration dans le génome eucaryote. Des travaux récents montrent clairement que cette étape de rétrotranscription peut avoir lieu au sein même du virion, lorsqu'il est encore dans sa phase extracellulaire. Ainsi, jusqu'à 2% des particules virales, dans le cas du liquide séminal, peuvent avoir terminé leur rétrotranscription avant de fusionner avec la cellule cible. Il est donc indispensable que les inhibiteurs de la transcriptase inverse (TI) puissent pénétrer le virion avant qu'il ait atteint la cellule.

Les études de Perelson et al. (1996, Science 271.1582-1586) sur la dynamique virale montrent que la durée de vie moyenne d'une particule virale dans le plasma sanguin est de l'ordre de 8 heures soit presque le quart de la durée de vie estimée du VHI-1 in vivo. Le virion, dans sa phase extracellulaire est donc une cible potentielle pour ces inhibiteurs.

On notera que d'autres thiopyridinones, ne présentant pas d'activité anti-HIV-1 ont été décrites.

Ainsi, l'article de CROISY-DELCEY et al.(1983 J.Med. Chem. vol.26, n°9, 1329-1333) décrit la synthèse d'analogues du lucanthone qui présentent des activités antitumorales et bactéricides. Les composés intermédiaires 7b, 17a et 17b sont des pyridinones.

L'article de RIVALLE et al.((1980) J. Hétérocycl. Chem., vol. 17, n°2, 245-248) décrit la synthèse de pyridoquinoléines, par l'intermédiaire de composés dont certains sont des pyridinones. Aucune activité n'est mentionnée, que ce soit pour les composés finaux ou intermédiaires.

L'article de UPTON ((1986) J. Chem. Soc. Perkin Trans. 1, n°7, 1225-1229), décrit la synthèse d'anthracènes par l'intermédiaire en particulier d'hydroxypyridines, et non de pyridinones.

Le demandeur s'est attaché à trouver des nouvelles molécules présentant une forte activité inhibitrice, c'est-à-dire actives à faibles doses, une faible cytotoxicité et pénétrant la particule virale.

Il a montré que des 4-aryl-thio-pyridin-2(1H)-ones substituées présentent une forte activité inhibitrice et une faible cytotoxicité tout en pénétrant la particule virale.

La présente invention a donc pour objet des composés de formule (3), représentée sur la figure 3, dans laquelle:
- R₁ et R₂ représentent indépendamment un atome d'hydrogène, un groupe aliphatique ou un groupe alkyloxyalkyle dans lequel les chaînes alkyle sont en C₁ à C₄, ou forment ensemble un cycle aromatique,
- R₃ représente un groupe NH₂, NHCOCH₃, NO₂ ou COOC₂H₅.
- R₄ représente un groupe phényle, pyrimidine, benzimidazole, benzoxazole, benzothiazole, thiazoline, imidazole ou pyridine éventuellement substitué par un ou plusieurs groupes aliphatiques et/ou par un ou plusieurs groupes hydroxy.

De manière avantageuse, R₁ et R₂ représentent indépendamment un atome d'hydrogène, un groupe alkyle en C₁ à C₄, ou un groupe alkyloxyméthyle, préférentiellement éthoxyméthyle.
R₆ peut être un groupe alkyle en C₁ à C₆ éventuellement substitué par un groupe phényle, ou R₆ peut être un groupe benzyle.
R₇ peut être un groupe alkyle en C₁ à C₆, en particulier un groupe éthyle.
R₄ est avantageusement un groupe phényle substitué par des groupes alkyle, préférentiellement par deux groupes méthyle, et encore plus préférentiellement un groupe phényle substitué en positions méta par deux groupes méthyle.

Des composés particulièrement avantageux selon la présente invention sont les composés suivants:
la 5-éthyl-6-méthyl-3-nitro-4-(3',5'-diméthylphényl) thio-pyridin-2(1H)-one (formule 7c),
la 5-éthoxyméthyl-3-nitro-4-(3',5'-diméthylphényl)thio-pyridin-2(1H)-one (formule 7g),
la 3-amino-5-éthyl-6-méthyl-4-(3',5'-diméthylphényl thio-pyridin-2(1H)-one (formule 8c),
la 3-amino-5-méthyl4-(3',5'-diméthylphényl)thio-pyridin-2(1H)-one (formule **8a**),
la 3-amino-5-éthyl-4-(3',5'-diméthylphényl)thio-pyridin-**2**(1H)-one (formule 8b),
la 3-amino-5-éthoxyméthyl-4-(3',5'-diméthylphényl)thio-pyridin-2(1H)-one (formule 8g),
la 5-éthyl-6-méthyl-3-carbéthoxy-4-(3',5'-diméthylphényl)thio-pyridin-2(1H)-one (formule 15).

La présente invention concerne également l'obtention des composés selon l'invention. Ainsi les composés dans lesquels R₃ représente NO₂ (formules 7 et 9), peuvent être obtenus en faisant réagir une chloronitropyridinone de formule (6), avec un thio phénol, ou un dérivé mercapto d'un hétérocycle éventuellement substitué de formule R₄SH.

La nitropyridinone obtenue peut être réduite par du chlorure stanneux hydraté ou par hydrogénation catalytique pour la préparation de composés dans lesquels R₃ représente NH₂ (formules 8 et 10).

Ainsi, les composés de formules 7 et 8, selon l'invention, ont été synthétisés selon le schéma réactionnel représenté sur la figure 4, à partir des hydroxypyridin-2(1H)-ones correspondantes (composés 4).

Les composés 9a à 9f ont été obtenus selon le schéma représenté sur la figure 5.

Les composés de formule 4 sont quant à eux obtenus soit comme décrit par Legraverend et al. (Nucleosides and Nucleotides, 1986, 5(2), 125-134) ou par réaction de l'éthyl-2-éthyl-3-aminocrotonate (composé 12) avec du malonate de diéthyle afin d'obtenir le composé intermédiaire 13 qui est lui-même hydrolysé par de l'acide chlorhydrique.

Les nitropyridinones (composés 5) ont été préparés par réaction avec de l'acide nitrique HNO₃ puis ont été transformés en chloronitropyridinones (composés 6) comme décrit par C.H. Nguyen et al. (Anti-Cancer Drug Design, 1992, 7, 219-233) par réaction des composés de formule (5) avec un mélange POCl₃/chlorure de benzyltriéthylammonium/CH₃CN chauffé au reflux.

La condensation du composé (6a) avec le 3,5-diméthylthiophénol, effectuée en présence de triéthylamine dans l'éthanol à la température ambiante, conduit à la 4-(3',5'-diméthylphényl)thio-5-méthyl-3-nitropyridine-2(1H)-one (composé 7a). La réduction avec du chlorure stanneux dihydraté dans de l'acétate d'éthyle à ébullition conduit à l'amine de formule 8a.

Les composés 7d, 7e et 8e ont été obtenus par condensation de la chloronitropyridinone (6a) avec du thiophénol ou du m-thiocrésol, suivie de la réduction de la fonction nitro (dans le cas du composé 8e).

L'aminopyridinone (10) a été obtenue par réduction du composé 9d, en présence de chlorure stanneux dihydraté et d'acétate d'éthyle à reflux.

Les composés dans lesquels R₃ représente NHCOR₆ et R₄ représente un groupe phényle éventuellement substitué, peuvent être obtenus en faisant réagir un composé de formule (R₆CO)₂O ou R₆COZ, dans lequel Z est un radical susceptible de se libérer et de permettre la formation d'une liaison amide, avec une aminopyridinone.

Ainsi, les amides 11a à 11e et 11g ont été synthétisés selon le schéma réactionnel représenté sur la figure 6 par modification de la fonction amino de la 3-amino-5-méthyl-4-(3',5' diméthylphényl)-thio-pyridin-2(1H)-one (composé 8a) ou de la 3-amino-5-éthyl-6-méthyl-4-(3',5'diméthylphényl)thio-pyridin-2(1H)-one (composé 8c). La réaction a été effectuée en présence de formiate d'éthyle, d'anhydride acétique, de chlorure de propionyle, d'anhydride heptanoïque ou de chlorure de phénylacétyle.

Le dérivé 3-N-(éthoxycarbamyle) (composé 11f) a été synthétisé dans des conditions similaires, avec du chloroformiate d'éthyle.

Les 5-éthyl-pyridinone et 5-éthyl-6-méthyl-pyridinone ont été obtenues soit en partant de la 5-éthyl-4-hydroxy-pyridin-2(1H)-one (composé 4b) soit de la 5-éthyl-4-hydroxy-6-méthyl-pyridin-2(1H)-one (composé 4c). La figure 7 illustre le schéma réactionnel pour l'obtention du composé 4c par une succession de deux étapes à partir de l'éthyl-2-éthylaminocrotonate (composé 12). La nitration, la monochloration, la substitution avec le 3,5-diméthylthiophénol et la réduction de la fonction nitro s'effectuent ensuite selon le schéma réactionnel de la figure 4, conduisant aux dérivés 3-nitro et 3-amino-pyridinones 7b et 8b et 7c et 8c respectivement. On a également obtenu de la même manière les analogues benzo(e)pyridinones 7f et 8f en utilisant comme composé de départ la quinoléine 2,4-diol (composé 4f) disponible dans le commerce.

Les composés dans lesquels R₃ représente un groupe COOR₇ et R₄ représente un groupe phényle éventuellement substitué ont été obtenus en faisant réagir une 4-chloropyridinone de formule 14 avec un thiophénol éventuellement substitué selon le schéma de la figure 7. La 4-chloropyridinone de formule 14 peut quant à elle être obtenue par réaction de l'hydroxypyridinone de formule 13 en présence de POCl₃, selon le schéma de la figure 7.

Ainsi la 3-carbéthoxy-5-éthyl-4-hydroxy-5-méthylpyridine-2(1H)-one (composé 13), obtenue comme produit intermédiaire au cours de la préparation de la pyridinone (4c), a été transformée successivement en 4-chloropyridinone (composé 14) et en 4-phénylthiopyridinone (composé 15). L'hydrolyse de la 3-carbéthoxypyridinone 15 conduit, par décarboxylation, à la pyridinone (composé 16) non substituée en position 3.

L'obtention des composés selon l'invention n'est pas limitée aux procédés décrits ci-dessus. Ils peuvent être obtenus par tous les moyens connus de l'homme du métier.

La présente invention a de plus pour objet des composés, intervenant dans la synthèse des composés de formule (3) dans laquelle R₁ et R₂ sont indépendamment des groupes éthyle ou méthyle et R₃ est NO₂ ou NH₂, répondant à la formule (6) dans laquelle R₁ et R₂ ont ces natures.

Elle est de plus relative à des composés, intervenant dans la synthèse des composés de formule (3) dans laquelle R₁ et R₂ sont indépendamment des groupes éthyle et méthyle et R₃ est le groupe CO₂C₂H₅, répondant à la formule (17) représentée sur la figure 8 dans laquelle R₁ et R₂ ont ces natures.

La présente invention est en outre relative à des compositions pharmaceutiques contenant une quantité efficace d'un composé selon l'invention tel que décrit ci-dessus, en mélange avec des excipients compatibles.

Elle a aussi pour objet un médicament contenant lesdits composés.

Elle est enfin relative à l'utilisation d'un de ces composés pour la fabrication d'un médicament pour le traitement des maladies liées aux VIH.

Bien que les présents composés puissent être administrés par toute voie connue de l'homme du métier, ils sont préférentiellement administrés aux patients par voie orale, parentérale (incluant les injections sous cutanées, intraveineuses, intramusculaires, intrasternales), rectale et par inhalation.

Les compositions selon la présente invention sont formulées pour répondre aux exigences des voies d'administration précitées et par exemple présentées sous forme de comprimés, de gélules, de solutions stériles injectables, par exemple de suspensions aqueuses stériles injectables ou de suspensions huileuses, de préparations nasales ou de suppositoires.

Quand elles sont administrées de manière orale, sous forme de suspensions, ces compositions peuvent être préparées selon les méthodes connues de l'homme du métier, et peuvent contenir de la cellulose microcristalline comme charge, de l'acide alginique ou de l'alginate de sodium comme agent de suspension, de la méthylcellulose comme agent améliorant la viscosité, et des agents aromatisants.

Quand elles sont administrées par voie nasale, ou par inhalation, ces compositions peuvent être sous la forme de solutions salines, et contenir de l'alcool benzylique, ou tout autre conservateur adapté, des substances destinées à améliorer l'absorption, afin d'améliorer la biodisponibilité, des fluorocarbones, et tout autre agent de dispersion et de solubilisation connu de l'homme du métier.

Les solutions injectables, ou les suspensions peuvent être formulées en utilisant des diluants ou des solvants non toxiques, acceptables par voie parentérale, tel que le manitol, le 1,3-butanediol, de l'eau, de la solution de Ringer ou une solution de chlorure de sodium isotonique ou tout autre agent de dispersion de mouillage ou de suspension, telles que des huiles stériles en particulier les diglycérides ou les monoglycérides synthétiques et les acides gras tels que l'acide oléique.

Quant elles sont administrées sous forme rectale, c'est-à-dire sous forme de suppositoires, ces compositions peuvent être préparées par mélange d'un composé selon l'invention avec un excipient adapté non irritant, tel que du beurre de cacao, des esters synthétiques de glycérol, ou des polyéthylèneglycoles, qui sont solides aux températures ordinaires, mais qui se liquéfient ou qui se dissolvent dans la cavité rectale afin de relarguer le composé.

Les composés selon la présente invention peuvent être administrés oralement dans des quantités comprises entre 1 et 100 mg/kg corporel. Néanmoins les doses spécifiques, et les fréquences du traitement varient de patient à patient et peuvent dépendre de différents facteurs incluant l'activité du composé employé, sa stabilité métabolique, sa rapidité d'action, ainsi que l'âge, le poids et l'état général du patient au moment de l'administration, le taux d'excrétion, les autres médicaments utilisés, et tout autre facteur inhérent au patient traité.

Les compositions selon la présente invention sont destinées à inhiber la transcriptase inverse des VIH, à la prévention et au traitement de l'infection par les VIH, et au traitement des conséquences pathologiques d'une telle infection, tel que le SIDA. Le traitement du SIDA, ou la prévention ou le traitement de l'infection par les VIH peut être définie, sans pour autant que cette définition soit limitative, comme le traitement d'une gamme importante d'état de l'infection par les VIH, tel que le SIDA ou l'ARC (Aids Related Complex).

La présente invention est illustrée sans pour autant être limitée par les exemples qui suivent.

Les figures suivantes illustrent l'invention :
Les figures 1 à 3 représentent respectivement les formules des composés (1), (2) et (3).
Les figures 4 à 7 représentent des schémas réactionnels pour l'obtention des composés selon l'invention;
La figure 8 représente la formule du composé (17).
La figure 9 illustre l'effet du composé 7c sur l'activité des transcriptases inverses des virus VIH-1 et VIH-2, en présence de deux couples matrice-amorce;
La figure 10 illustre l'effet dose du composé 7c sur l'inhibition de la transcriptase inverse du VIH-1. L'ADN complémentaire attendu, long de 147 nucléotides, est indiqué à gauche du gel (puits 1). Les puits 2 à 5 correspondent à des concentrations décroissantes du composé 7c (puits 2: 400nM, puits 3: 300 nM, puits 4: 100 nM et puits 5: 40 nM). Le puits 6 ne contient pas de composé 7c.
La figure 11 est un schéma de la sonde utilisée pour mettre en évidence l'ADN complémentaire de la figure 10;
La figure 12 est une représentation double-réciproque (Linewear-Burk) de l'inhibition de la transcriptase inverse du VIH-1 par le composé 7c avec le poly C-oligo dG comme couple matrice-amorce et dGTP comme substrat;
La figure 13 est une représentation double-réciproque , similaire à la figure 9, dans laquelle le poly A-oligo dT est utilisé comme couple matrice-amorce et le dTTP comme substrat;
La figure 14 représente la mesure de l'activité transcriptase inverse (Ti) après filtration sur anopore;
La figure 15 représente la mesure de l'infectiosité après filtration sur anopore.

### Caractérisation des produits:

On a effectué une chromatographie sur couche mince (CCM) sur des plaques préalablement recouvertes de gel de silice 6OF254 (Merck). Pour révéler les composés, on a exposé les plaques de CCM à la lumière UV. On a procédé à des purifications sur des colonnes de gel de silice (40-60 µm) par chromatographie à moyenne pression. Tous les points de fusion ont été mesurés sur un appareil Electrothermal 9200 et n'ont pas été corrigés. On a enregistré les spectres RMN-¹H dans les solvants donnés à l'aide d'un appareil Bruker AC 200 avec comme étalons internes du CHCI₃ (δ = 7,25 ppm) ou du DMSO (δ = 2,54 ppm) (*,# = attributions interchangeables). Les analyses élémentaires (tableau 1) ont été effectuées par le Service Central de Microanalyses du CNRS, 91190 Gif-sur-Yvette, France.

### EXEMPLE 1:

### Préparation de l'éthyl-2-éthyl-3-aminocrotonate (composé 12).

On dissout de l'éthyl-2-éthylacétoacétate (150 g, 0,95 mole) et du nitrate d'ammonium (84 g, 1,04 moles) dans 1,1 I de tétrahydrofurane anhydre. Le mélange est agité pendant 5 jours avec un barbotage d'ammoniac. On évapore le solvant à la température ambiante et on ajoute 1 I d'eau. Le mélange est agité pendant 30 mn. On isole le résidu solide incolore par filtration et on le recristallise dans l'hexane pour donner le produit **12** (107 g, 72%) sous forme de cristaux incolores: point de fusion 61°C; RMN-¹H (CDCl₃) δ 4,11 (2H, q, J =7hz, O*CH*_{*2*}CH₃), 2,17 (2H, q, J =7Hz, *CH*_{*2*}CH₃), 1,93 (3H, s, CH₃), 1,24 (3H, t, J = 7Hz, OCH₂*CH*_{*3*}), 0,94 (3H, t, J = 7Hz, CH₂*CH*_{*3*}). Anal. C₈H₁₅NO₂ (C,H,N).

### EXEMPLE 2:

### Préparation de la 4-hydroxy-5-éthyl-6-méthyl-3-carbéthoxy-pyridin-2(1H)-one ( composé 13).

On dissout lentement du sodium (48,34 g, 2,10 moles, en morceaux dans du kérosène) dans 530 ml d'éthanol sous atmosphère d'azote pendant 3 h. On chauffe le mélange à reflux et on ajoute goutte à goutte du malonate de diéthyle (335 ml, 2,20 moles, fraîchement distillé) pendant 30 mn. Toujours à reflux, on ajoute goutte à goutte l'aminocrotonate ( **composé 13**) (150 g, 0,96 mole) dans 200 ml d'éthanol. Le mélange est agité à reflux pendant 72 h pour donner une suspension jaune pâle. On refroidit cette suspension à la température ambiante et on isole le précipité par filtration. On dissout le solide dans l'eau, on le refroidit à 0°C et on l'acidifie avec une solution aqueuse d'acide chlorhydrique jusqu'à pH 1. On isole le précipité par filtration, on le lave avec de l'eau et on le cristallise dans du toluène pour donner le produit **13** (109,6 g, 51%) sous forme de cristaux blancs: point de fusion 196-197°C; RMN-¹H (DMSO-*d*₆) δ 11,52 (1H, s, NH*-1), 11,32 (1H, s, OH*), 4,34 (2H, q, J= 7Hz, COO*CH*_{*2*}CH₃), 2,39 (2H, q, J= 7,5 Hz, *CH*_{*2*}CH₃), 2,23 (3H, s, CH₃-6), 1,31 (3H, t, J = 7Hz, COOCH₂*CH*_{*3*}), 1,02 (3H, t, J = 7Hz, CH₂*CH*_{*3*}). Anal. C₁₁H₁₅NO₄ (C,H,N).

### EXEMPLE 3:

### Préparation de la 5-éthyl-4-hydroxy-6-méthyl-pyridin-2(1H)-one ( composé 4c).

On dissout l'ester (13) préparé dans l'exemple 2 (17,2 g, 76,4 mmoles) dans 1,2 I d'une solution aqueuse de HCI 1N et on chauffe le mélange à reflux pendant 36 h. Après évaporation du solvant, on ajoute 100 ml d'eau et on neutralise le mélange avec une solution aqueuse d'ammoniac. On isole le précipité par filtration et on le lave avec de l'eau. On obtient le produit **4c** (11,2 g, 96%) sous forme d'un solide blanc: point de fusion 360°C; RMN-¹H (DMSO-*d*₆) δ 10,75 (1H, s, NH-1), 5,46 (1H, s, H-3), 2,32 (2H, q, J = 7Hz, *CH*_{*2*}CH₃), 2,13 (3H, s, CH₃-6), 0,99 (3H, t, J = 7Hz, CH₂*CH*_{*3*}). Anal. C₈H₁₁N₁O₂(C,H,N).

### EXEMPLE 4:

### Préparation de la 5-éthyl-4-hydroxy-3-nitropyridin-2(1H)-one (composé 5b).

Une suspension de **composé 4b** obtenue comme décrit par Legraverend et al. (Nucleosides and Nucleotides, 1986, 5(2), 125-134) (5,00 g, 36,0 mmoles) dans 40 ml d'acide nitrique (d = 1,33) est agitée pendant 10 mn à la température ambiante et à 75°C pendant 12 mn. On ajoute immédiatement 150 ml d'eau glacée, on isole le précipité jaune par filtration et on le recristallise dans l'eau pour donner la nitropyridinone **5b** (5,4 g, 82%) sous forme de cristaux jaunes: point de fusion 213-214 °C; RMN-¹H (DMSO-*d*₆) δ 11,85 (1H, s, NH-1), 7,39 (1H, s, H-6), 2,42 (2H, q, J= 7Hz, CH₂), 1,10 (3H, t, J= 7Hz, CH₃). Anal. C₇H₈N₂O₄.0.25H₂O(C,H,N).

### EXEMPLE 5:

### Préparation de la 5-éthyl-4-hydroxy-6-méthyl-3-nitropyridin-2(1H)-one ( composé 5c).

Comme décrit pour le composé **5b** à l'exemple 4, une suspension du composé **4c** (4,00 g, 26,0 mmoles) dans 32 ml d'acide nitrique (d = 1,33) est agitée à la température ambiante pendant 10 mn et à 80°C pendant 10 mn. On ajoute immédiatement 80 ml d'eau glacée, et on isole le précipité par filtration et on le recristallise dans l'eau pour donner la nitropyridinone **5c** (4,4 g, 85%) sous forme de cristaux jaunes: point de fusion 255-256 °C; RMN-¹H (OMSO-*d*₆) δ 11,90 (1H, s, NH-1), 2,47 (2H, q, J = 7Hz, *CH*_{*2*}CH₃), 2,27 (3H, s,CH₃-6), 1,03 (3H, t, J=7Hz, CH₂*CH*_{*3*}). Anal. C₈H₁₀N₂O₄ (C,H,N).

### EXEMPLE 6:

### Préparation de la 4-hydroxy-3-nitroguinolin-2(1H)-one (composé 5f).

Comme décrit pour le composé **5b** à l'exemple 4, une suspension du composé **4f** (9,00 g, 55,8 mmoles) dans 70 ml d'acide nitrique (d = 1,33) est agitée à la température ambiante pendant 15 mn et à 80°C pendant 15 mn. On ajoute immédiatement 250 ml d'eau glacée, on isole par filtration le précipité jaune, on le lave avec de l'eau et on le sèche sur P₂O₅ sous vide pour donner la nitropyridinone **5f** (11,3 g, 98%) sous forme d'un solide jaune: point de fusion 250°C; RMN-¹H (DMSO-*d*₆) δ 11,85 (1H, s, NH-1), 8,06 (1H, d, J=8Hz, H-8), 7,68 (1H, td, J₁= 8Hz, J₂= 1Hz, H-5), 7,37-7,26 (2H, m, H-6 et 7). Anal. C₉H₆N₂O₄(C,H,N).

### EXEMPLE 7:

### Préparation de la 4-chloro-5-éthyl-3-nitropyridin-2(1H)-one (composé 6b).

On ajoute de l'oxychlorure de phosphore (6,7 ml, 39,1 mmoles) à une solution de composé **5b** (3,00 g, 16,3 mmoles) et de chlorure de benzyltriéthylammonium (14,90 g, 65,2 mmoles) dans l'acétonitrile (60 ml). Le mélange obtenu est agité à 40°C pendant 30 mn et chauffé à reflux pendant 1 h. Après évaporation du solvant, on ajoute 60 ml d'eau et on agite le mélange à la température ambiante pendant 3 h. On recueille le précipité jaune, on le lave avec du cyclohexane (3 x 6 ml) et on le sèche pour donner **le composé 6b** (2,4 g, 74%) sous forme de cristaux jaunes pâles: RMN-¹H (DMSO-*d*₆) δ 13,19 (1H, s, NH-1), 7,73 (1H, s, H-6), 2,56 (2H, q, J = 7,5 Hz, *CH*_{*2*}CH₃), 1,16 (3H, t, J =7,5 Hz, CH₂*CH*_{*3*}).

### EXEMPLE 8:

### Préparation de la 4-chloro-5-éthyl-6-méthyl-3-nitropyridin-2(1H)-one (composé 6c).

Comme décrit pour le composé **6b** à l'exemple 7, on ajoute de l'oxychlorure de phosphore (8,3 ml, 88,8 mmoles) à une solution de **composé 5c** (4,00 g, 20,2 mmoles) et de chlorure de benzyltriéthylammonium (18,40 g, 80,8 mmoles) dans l'acétonitrile (80 ml). Le mélange obtenu est agité à 40°C pendant 30 mn et chauffé à reflux pendant 1 h. Après évaporation du solvant, on ajoute 150 ml d'eau et on agite le mélange à la température ambiante pendant 48 h. On recueille le précipité brun, on le lave avec du cyclohexane (3 x 10 ml) et on le recristallise dans l'éthanol pour donner le composé **6c** (1,5 g, 34%) sous forme de cristaux jaunes pâles: RMN-¹H (DMSO-*d*₆) δ 12,98 (1H, s, NH-1), 2,58 (2H, q, J=7,5Hz,*CH*_{*2*}CH₃), 2,38 (3H, s, CH₃-6), 1,08 (3H, t, J=7,5Hz, CH₂*CH*_{*3*}).

### EXEMPLE 9:

### Préparation de la 4-chloro-3-nitro-2-quinoléin-2(1H)-one (composé 6f).

Comme décrit pour le composé **6b** à l'exemple 7, on ajoute de l'oxychlorure de phosphore (4,0 ml, 46,6 mmoles) à une solution de **composé 5f** (4,00 g, 19,4 mmoles) et de chlorure de benzyltriéthylammonium (17,68 g, 77,6 mmoles) dans l'acétonitrile (80 ml). Le mélange obtenu est agité à 45°C pendant 30 mn sous atmosphère d'azote et chauffé à reflux pendant 20 mn. Après évaporation du solvant, on ajoute 120 ml d'eau glacée. Le mélange est neutralisé avec 12 ml d'une solution aqueuse diluée d'ammoniac (14%) à 0°C et agité à cette température pendant 1 h. Le précipité jaune est recueilli et agité dans 120 ml d'hexane pendant 1 h. Après filtration, on sèche le composé **6f** (2,9 g, 67%) sur P₂O₅ sous vide pour donner un solide jaune: RMN-¹H (DMSO-*d*₆) δ 12,10 (1H, s, NH-1), 8,04 (1H, d, J=8Hz, H-8), 7,84 (1H,t,J=8Hz,H-5), 7,54-7,43(2H,m,H-6 et 7).

### EXEMPLE 10:

### Préparation de la 5-méthyl-3-nitro-4-(3',5'-diméthylphényl)thiopyridin-2(1H)-one (composé 7a).

Un mélange du composé **6a** obtenu selon Nguyen et al. (1992, précédemment cités) (1,88 g, 10,0 mmoles) dans 20 ml d'éthanol et 2 ml de triéthylamine est agité jusqu'à homogénéité. On ajoute goutte à goutte du 3,5-diméthylthiophénol (1,39 g, 10,1 mmoles). Après 1 h sous agitation à la température ambiante, on isole le précipité par filtration et on le lave avec du cyclohexane (20 ml). On obtient le produit **7a** (2,66 g, 92%) sous forme d'un solide jaune: point de fusion 235°C; RMN-¹H (DMSO-*d*₆) δ 12,27 (1H, s, NH-1), 7,63 (1H, s, H-6), 7,01 (1H, s, H-4'), 6,96 (2H, s,H-2' et 6') 2,27 (6H, s, CH₃-3' et 5'), 1,89 (3H, s, CH₃-5). Anal. C₁₄H₁₄N₂O₃S.0,25H₂O (C,H,N,S).

### EXEMPLE 11:

### Préparation de la 5-éthyl-3-nitro-4-(3',5'-diméthylphényl)thio-pyridin-2(1H)-one (composé 7b).

Comme décrit pour le composé **7a** à l'exemple 10, un mélange du composé **6b** (2,42 g, 11,9 mmoles) dans 20 ml d'éthanol et 2 ml de triéthylamine est agité jusqu'à homogénéité. On ajoute goutte à goutte du 3,5-diméthylthiophénol (1,64 ml, 12,1 mmoles). Après 4 h sous agitation à la température ambiante, on isole le précipité par filtration et on le recristallise dans de l'éthanol (15 ml). On obtient le produit **7b** (1,94 g, 53%) sous forme de cristaux jaunes: point de fusion 197°C; RMN-¹H (DMSO-*d*₆) δ 12,29 (1H, s, NH-1),7,59 (1H, s, H-6), 7,00 (1H,s,H-4'), 6,94 (2H, s, H-2' et 6') 2,36 (2H, q, J=8Hz, *CH*_{*2*}CH₃), 2,26 (6H, s, CH₃-3' et 5'), 1,03 (3H, t, J = 7Hz, CH₂*CH*_{*3*}). Anal. C₁₅H₁₆N₂O₃S (C,H,N,S).

### EXEMPLE 12:

### Préparation de la 5-éthyl-6-méthyl-3-nitro-4-(3',5'-diméthylphényl)thio-pyridin-2(1H)-one (composé 7c).

Comme décrit pour le composé **7a** à l'exemple 10, un mélange du composé **6c** (0,90 g, 4,1 mmoles) dans 16 ml d'éthanol et 9 ml de triéthylamine est agité jusqu'à homogénéité. On ajoute goutte à goutte du 3,5-diméthylthiophénol (0,57 ml, 4,2 mmoles). Après 3 h sous agitation à la température ambiante, on isole le précipité par filtration et on le cristallise dans l'éthanol (50 ml). On obtient le produit **7**c (1,07 g, 81%) sous forme de cristaux jaunes: point de fusion 236-237°C; RMN-¹H (DMSO-*d*₆) δ 12,28 (1H, s, NH-1), 6,98 (1H, s, H-4'), 6,94 (2H, s, H-2' et 6') 2,26 (6H, s, CH₃-3' et 5'), 2,12 (3H,s,CH₃-6), 0,87 (3H, t, J = 7Hz, CH₂*CH*_{*3*}), le signal de *CH*_{*2*}CH₃ et le signal du DMSO se chevauchent. Anal. C₁₆H₁₈N₂O₃S (C,H,N,S).

### EXEMPLE 13:

### Préparation de la 5-méthyl-3-nitro-4-phénylthio-pyridin-2(1H)-one (composé 7d).

Comme décrit pour le composé **7a** à l'exemple 10, un mélange du composé **6a** (0,20 g, 1,1 mmoles) dans 2 ml d'éthanol et 0,2 ml de triéthylamine est agité jusqu'à homogénéité. On ajoute du thiophénol (0,12 g, 1,1 mmoles). Après 15 h sous agitation à la température ambiante, on isole le précipité jaune par filtration et on le lave avec de l'éthanol pour donner le produit **7d** (0,13 g, 47%) sous forme d'un solide jaune: point de fusion 214-216°C; RMN-¹H (DMSO-*d*₆) δ 12,86 (1H,s,NH-1), 7,65 (1H, s, H-6), 7,45-7,36 (5H, m, H-2',3',4',5' et 6') 1,89 (3H, s, CH₃-5). Anal. C₁₂H₁₀N₂O₃S.0,125H₂O (C,H,N,S).

### EXEMPLE 14:

### Préparation de la 5-méthyl-3-nitro-4-(3'-méthylphényl) thio-pyridin-2(1H)-one (composé 7e).

Comme décrit pour le composé **7a** à l'exemple 10, un mélange du composé **6a** (0,50 g, 2,6 mmoles) dans 10 ml d'éthanol et 0,3 ml de triéthylamine est agité jusqu'à homogénéité. On ajoute goutte à goutte du *m*-thiocrésol (0,35 g, 2,8 mmoles) dans 5 ml d'éthanol. Après 15 h sous agitation à la température ambiante, on évapore le solvant et on ajoute 15 ml d'eau. On agite la suspension pendant 2 h à la température ambiante. Le précipité jaune est isolé par filtration, lavé avec 6 x 2 ml d'eau, séché et recristallisé dans l'éthanol pour donner le produit **7e** (0,52 g, 71%) sous forme d'aiguilles jaunes: point de fusion 222-223°C; RMN-¹H (DMSO-*d*₆) δ 12,85 (1H, s, NH-1), 7,64 (1H, s, H-6), 7,27(1H, t, J = 8Hz, H-5'), 7,20-7,14 (3H, m, H-2',4' et 6'), 2,31 (3H, s, CH₃-3'), 1,89 (3H, s, CH₃-5). Anal. C₁₃H₁₂N₂O₃S.0,25H₂O (C,H,N,S).

### EXEMPLE 15:

### Préparation de la 3-nitro-4-(3',5'-diméthylphényl)-thio-quinoléin-2(1H)-one ( composé 7f).

Comme décrit pour le composé **7a** à l'exemple 10, un mélange du composé **6f** (1,40 g, 6,2 mmoles) dans 50 ml d'éthanol et 1,3 ml de triéthylamine est agité jusqu'à homogénéité. On ajoute goutte à goutte, pendant 15 mn, du 3,5-diméthylthiophénol (0,86 ml, 6,3 mmoles) dans 30 ml d'éthanol. Le récipient est équipé d'un piège contenant du CaCl₂. Après 2 h 30, sous agitation à 50°C, on évapore le solvant et on ajoute 100 ml d'eau. On neutralise le mélange avec une solution aqueuse diluée d'ammoniac et on agite à la température ambiante pendant 1 h. On isole le précipité brun par filtration, on le lave avec de l'hexane et on le cristallise dans l'éthanol (200 ml). On obtient le produit **7f** (1,19 g, 59%) sous forme de cristaux jaune-orangés: point de fusion 294°C; RMN-¹H (DMSO-*d*₆) δ 12,89 (1H, large, NH-1), 7,96 (1H, dd, J₁ = 8Hz, J₂ = 1Hz, H-8), 7,70 (1H, td, J₁ = 8Hz, J₂ = 1Hz, H-5), 7,48 (1H, d, J = 8Hz, H-6*), 7,31 (1H, td, J₁ = 8 Hz ,J₂ = 1Hz, H-7*), 7,05 (2H, s, H-2' et 6'), 6,98 (1H, s, H-4'), 2,23 (6H, s, CH₃-3' et 5'). Anal. C₁₇H₁₄N₂O₃S (C,H,N,S).

### EXEMPLE 16:

### Préparation de la 5-éthoxyméthyl-3-nitro-4-(3',5'-diméthylphényl)thio-pyridin-2(1H)-one (composé 7g):

Comme décrit pour le composé 7a à l'exemple 10, un mélange du composé 6g obtenu selon C.H. Nguyen et al. (1992, précédemment cités) (0,15 g, 0,6 mmole) dans 5 ml d'éthanol et 0,1 ml de triéthylamine est agité jusqu'à homogénéité. On ajoute goutte à goutte du 3,5-diméthylthiophénol (0,09g, 0,6 mmole) dans 3 ml d'éthanol. Après 2 h sous agitation à la température ambiante, on évapore le solvant et on ajoute 10 ml d'eau. On basifie le mélange par addition de 0,5 ml d'une solution aqueuse diluée d'ammoniac (14%) et on agite la suspension à la température ambiante pendant 2h. On isole le précipité jaune par filtration, on le lave avec de l'eau et de l'hexane et on le sèche pour donner le produit 7 g (0,16g, 74%) sous forme d'une poudre couleur crème: point de fusion 143-144°C; RMN-¹H (DMSO-d₆) δ 12,25 (1 H, S, NH-1), 7,75 (1H, S, H-6), 7,01 (1H, S, H-4'), 6,98 (2H, S, H-2' et 6'), 4,23 (2H, S, OCH₂-5), 3,37 (2H, q, J = 7H₂, CH₃CH₂O), 2,26 (6H, S, CH₃-3' et 5'), 1,08 (3H, t, J = 7H₂, CH₃CH₂O). Anal. C₁₆H₁₈N₂O₄S (C, H, N, S).

### EXEMPLE 17:

### Préparation de la 3-amino-5-méthyl-4-(3',5'-diméthylphényl)-thio-pyridin-2(1H)-one (composé 8a).

On ajoute du chlorure stanneux dihydraté (9,75 g, 43,0 mmoles) à une suspension de **composé 7a** (2,50 g, 8,6 mmoles) dans l'acétate d'éthyle (150 ml). On chauffe le mélange à reflux sous atmosphère d'argon pendant 1 h. Après refroidissement à 0°C, on ajoute de l'eau glacée (110 ml) et une solution saturée de carbonate de sodium jusqu'à l'obtention d'un pH basique. On élimine le précipité par filtration. On sépare les phases filtrées et on extrait la phase aqueuse avec de l'acétate d'éthyle. Les phases organiques rassemblées sont lavées avec une solution aqueuse saturée de chlorure de sodium (3 x 120 ml), séchées sur du sulfate de magnésium et évaporées. On purifie le résidu par chromatographie sur colonne en utilisant comme éluant un mélange dichlorométhane/éthanol (9:1) pour donner le produit **8a** (2,02 g, 87%) sous forme d'un solide jaune pâle: point de fusion 208°C; RMN-¹H (DMSO-*d*₆) δ 11,46 (1H, s, NH-1), 6,82 (1H, s, H-4'), 6,72 (2H, s, H-2' et 6'), 6,62 (1H, s, H-6), 5,46 (2H, s, NH₂-3), 2,22 (6H, s, CH₃-3' et 5'), 1,97 (3H, s, CH₃-5). Anal. C₁₄H₁₆N₂OS (C,H,N,S).

### EXEMPLE 18:

### Préparation de la 3-amino-5-éthyl-4-(3',5'-diméthylphényl)thio-pyridin-2(1H)-one (composé 8b).

Comme décrit pour le composé 8a à l'exemple 17, on ajoute du chlorure stanneux dihydraté (6,67 g, 29,6 mmoles) à une suspension du composé **7b** (1,80 g, 5,9 mmoles) dans l'acétate d'éthyle (100 ml). On chauffe le mélange à 70°C sous atmosphère d'argon pendant 1 h. Après refroidissement à 0°C, on neutralise la solution avec une solution saturée de carbonate de sodium (90 ml). On élimine le précipité par filtration. On sépare les phases filtrées et on extrait la phase aqueuse avec du dichlorométhane. Les phases organiques rassemblées sont lavées avec une solution aqueuse saturée de chlorure de sodium (3 x 120 ml), séchées sur du sulfate de magnésium et évaporées. On purifie le résidu par chromatographie sur colonne en utilisant comme éluant un mélange dichlorométhane/éthanol (9:1) pour donner le produit **8b** (1,04 g, 64%) sous forme d'un solide beige clair: point de fusion 208-209°C; RMN-¹H (DMSO-*d*₆) δ 11,51 (1H, s, NH-1), 6,81 (1H, s, H-4'), 6,68 (2H, s, H-2' et 6'), 6,56 (1H, s, H-6), 5,43 (2H, s, NH₂-3), 2,40 (2H, q, J = 7,5 Hz, *CH*_{*2*}CH₃), 2,21 (6H, s, CH₃-3' et 5'), 1,04 (3H, t, J = 7Hz, CH₂*CH*_{*3*}). Anal. C₁₅H₁₈N₂OS.0,25H₂O (C,H,N,S).

### EXEMPLE 19:

### Préparation de la 3-amino-5-éthyl-6-méthyl-4-(3',5'-diméthylphényl)thio-pyridin-2(1H)-one (composé 8c).

Comme décrit pour le composé **8a** à l'exemple 17, on ajoute du chlorure stanneux dihydraté (3,11 g, 13,2 mmoles) à une suspension de **composé 7c** (1,00 g, 3,1 mmoles) dans de l'acétate d'éthyle (50 ml). On chauffe le mélange à 70°C sous atmosphère d'argon pendant 1 h. Après refroidissement à 0°C, on neutralise la solution avec une solution saturée de carbonate de sodium (60 ml). On élimine le précipité par filtration. On sépare les phases filtrées et on extrait la phase aqueuse avec du dichlorométhane. Les phases organiques rassemblées sont lavées avec une solution aqueuse saturée de chlorure de sodium (3 x 120 ml), séchées sur du sulfate de magnésium et évaporées. On purifie le résidu par chromatographie sur colonne en utilisant comme éluant un mélange dichlorométhane/éthanol (95:5) et à la fin, de l'acétate d'éthyle pour donner le produit **8c** (0,80 g, 88%) sous forme d'un solide jaune pâle: point de fusion 188-189°C; RMN-¹H (DMSO-*d*₆) δ 11,52 (1H, s, NH-1), 6,80 (1H, s, H-4'), 6,70 (2H, s, H-2' et 6'), 5,17 (2H, s, NH₂-3), 2,21 (6H, s, CH₃-3' et 5'), 2,12 (3H, s, CH₃-6), 0,93 (3H, t, J = 7 Hz, CH₂*CH*_{*3*}), le signal de *CH*_{*2*}CH₃ et le signal du DMSO se chevauchent. Anal. C₁₆H₂₀N₂OS (C,H,N,S).

### EXEMPLE 20:

### Préparation de la 3-amino-5-méthyl-4-(3'-méthylphényl)thio-pyridin-2(1H)-one (composé 8e).

Comme décrit pour le composé **8a** à l'exemple 17, on ajoute du chlorure stanneux dihydraté (1,12 g, 5,0 mmoles) à une suspension du composé **7e** (0,36 g, 1,3 mmoles) dans l'acétate d'éthyle (25 ml). On chauffe le mélange à 80°C sous atmosphère d'argon pendant 20 h. Après refroidissement à la température ambiante, on neutralise la solution avec une solution saturée de carbonate de sodium (30 ml). La couleur jaune du mélange devient rouge. Après agitation pendant 15 mn, on élimine le précipité par filtration sur de la célite. On sépare les phases filtrées et on extrait la phase aqueuse avec du dichlorométhane. Les phases organiques rassemblées sont lavées avec une solution aqueuse saturée de chlorure de sodium, séchées sur du sulfate de sodium et évaporées. On purifie le résidu par chromatographie sur colonne en utilisant comme éluant un mélange acétate d'éthyle/heptane (2:1 à 4:1) pour donner le produit **8e** (0,18 g, 56%) sous forme d'une poudre brune: point de fusion 170-171°C; RMN-¹H (DMSO-*d*₆) δ 11,48 (1H, s, NH-1), 7,20 (1H, t, J=7,5 Hz, H-5'), 6,97-6,92 (3H, m, H-2', 4' et 6'), 6,62 (1H, s, H-6), 5,49 (2H, s, NH₂-3), 2,27 (3H, s, CH₃-3'), 1,97 (3H, s, CH₃-5). Anal. C₁₃H₁₄N₂O₃S (C,H,N,S).

### EXEMPLE 21:

### Préparation de la 3-amino-4-(3',5'-diméthylphényl)-thio-guinoléin-2(1H)-one (composé 8f).

Comme décrit pour le composé **8a** à l'exemple 17, on ajoute du chlorure stanneux dihydraté (1,04 g, 4,6 mmoles) à une suspension du composé **7f** (0,30 g, 0,9 mmole) dans de l'acétate d'éthyle (10 ml). On chauffe le mélange à 70°C pendant 1 h. Après refroidissement à 0°C, on ajoute 10 ml d'eau glacée et on basifie la solution avec une solution saturée de carbonate de sodium. On élimine le précipité jaune par filtration. On sépare les phases filtrées et on extrait la phase aqueuse avec 3 x 10 ml d'acétate d'éthyle. Les phases organiques rassemblées sont lavées avec une solution aqueuse saturée de chlorure de sodium (3 x 10 ml), séchées sur du sulfate de magnésium et concentrées. On cristallise le solide obtenu dans de l'acétate d'éthyle pour donner le produit **8f** (0,14 g, 52,5%) sous forme de cristaux verts: point de fusion 235-236°C; RMN-¹H (DMSO-*d*₆) δ 10,01 (1H, s, NH-1), 7,72 (1H, d, J = 8Hz, H-8), 7,33-7,09 (3H, m, H-5, 6 et 7), 6,80 (1H, s, H-4'), 6,77 (2H, s, H-2' et 6'), 6,06 (2H, s, NH₂), 2,18 (6H, s, CH₃-3' et 5'). Anal. C₁₇H₁₆N₂OS.0,25H₂O (C,H,N,S).

### EXEMPLE 22:

### Préparation de la 3-amino-5-éthoxyméthyl-4-(3',5'-diméthylphényl)thio-pyridin-2(1H)-one (composé 8g):

Le composé 7g (150 mg, 0,45 mmole) est dissous dans de l'éthanol absolu (5 ml). Le catalyseur (palladium sur charbon 10%, 50 mg) est additionné et le mélange est agité sous atmosphère d'hydrogène à la température ambiante pendant 24 heures. Le catalyseur est éliminé par filtration et le solvant est évaporé. Le résidu est purifié par chromatographie sur colonne en utilisant comme éluant un mélange dichlorométhane/éthanol (1: 0 à 96/4) pour donner le produit 8g (32 mg, 23%) sous forme de cristaux bruns: point de fusion 149-150°C; RMN-¹H (CDCl₃):12,27 (1H, S large, NH-1), 6,90 (1H, S, H-6), 6,76 (1H, S, H-4'), 6,73 (2H, S, H-2' et 6'), 4,91 (2H, S, NH₂-3), 4,35 (2H, S, CH₂O-5), 3,48 (2H, q, J = 7H₂, CH₃CH₂O), 2,22 (6H, S, CH₃-3' et 5'), 1,14 (3H, t, J = 7H₂, CH₃CH₂O). Anal. C₁₆H₂₀N₂O₂S₁ (C,H,N,S).

### EXEMPLE 23:

### Préparation de la 5-méthyl-3-nitro-4-(4',6'-diméthylpyrimidin-2-yl)thiopyridin-2(1H)-one (composé 9a).

Comme décrit pour le composé **7a** à l'exemple 10, un mélange du composé **6a** (0,14 g, 0,72 mmole) dans 10 ml d'éthanol et 0,26 ml de triéthylamine (1,80 mmoles) est agité jusqu'à homogénéité. On ajoute de la 2-mercapto-4,6-diméthylpyrimidine (0,09 g, 0,67 mmole). Après 3 h sous agitation au reflux, on évapore le solvant. On ajoute 10 ml d'eau et on agite la suspension à la température ambiante pendant 1 h. On isole le solide par filtration et on le sèche sur du P₂O₅ sous vide pour donner le produit **9a** (0,12 g, 58%) sous forme d'un solide jaune pâle: point de fusion 238-239°C; RMN-¹H (DMSO-*d*₆) δ 13,02 (1H, s, NH-1), 7,75 (1H, s, H-6), 7,15 (1H, s, H-5'), 2,36 (3H, s, CH₃-4' et 6'), 2,03 (3H, s, CH₃-5). Anal. C₁₂H₁₂N₄O₃S.0,125H₂O (C,H,N,S).

### EXEMPLE 24:

### Préparation de la 5-méthyl-3-nitro-4-(4'-hydroxy-6'-méthylpyrimidin-2-yl)thiopyridin-2(1H)-one (composé 9b).

Comme décrit pour le composé **7a** à l'exemple 10, un mélange du composé **6a** (0,50 g, 2,6 mmoles) dans 20 ml d'éthanol et 0,55 ml de triéthylamine (4,0 mmoles) est agité jusqu'à homogénéité. On ajoute de la 4-hydroxy-2-mercapto-6-méthylpyrimidine (0,38 g, 2,7 mmoles). La suspension est agitée à reflux pendant 4h30 et à la température ambiante pendant 15 h. On évapore le solvant et on ajoute 10 ml d'eau. On basifie le mélange avec une solution aqueuse diluée d'ammoniac et on agite pendant 2 h à la température ambiante. On élimine le précipité par filtration. On neutralise la phase aqueuse par addition d'une solution aqueuse d'acide chlorhydrique et on l'extrait avec 3 x 150 ml d'acétate d'éthyle. On concentre la phase organique et on purifie le résidu par chromatographie sur colonne en utilisant comme éluants un mélange hexane/acétate d'éthyle (1:1 à 0:1) et un mélange acétate d'éthyle/éthanol (95:5 à 9:1) pour donner le produit **9b** (0,23 g, 30%) sous forme de cristaux de couleur vert pâle: point de fusion >350°C; RMN-¹H (DMSO-*d*₆) δ 13,02 (1H, s, NH-1), 7,86 (1H, s, H-6), 6,04 (1H, s, H-5'), 2,22 (3H, s, CH₃-6'), 1,90 (3H, s, CH₃-5). Anal. C₁₁H₁₀N₄O₄S (C,H,N,S).

### EXEMPLE 25:

### Préparation de la 5-méthyl-3-nitro-4-(benzimidazol-2-yl)thio-pyridin-2(1H)-one (composé 9c).

Comme décrit pour le composé **7a** à l'exemple 10, un mélange du composé **6a** (0,10 g, 0,5 mmole) dans 10 ml d'éthanol et 0,11 ml de triéthylamine (0,8 mmole) est agité jusqu'à homogénéité. On ajoute du 2-mercaptobenzimidazole (0,08 g, 0,5 mmole) et on agite le mélange à reflux pendant 6h30 et à la température ambiante pendant 72 H. On évapore le solvant et on ajoute 10 ml d'eau. On agite la suspension à la température ambiante pendant 3 h. Le solide jaune obtenu est isolé par filtration, lavé à l'eau et séché sur P₂O₅ sous vide pour donner le produit 9**c** (0,11 g, 66%) sous forme de cristaux jaunes: point de fusion 272-275°C; RMN-¹H (DMSO-*d*₆) δ 12,93 (1H, s, NH-1), 7,72 (1H,s,H-6), 7,56 (2H,m,H-5' et 8'), 7,25-7,21 (2H,m,H-6' et 7'), 1,88 (3H,s,CH₃-5). Anal. C₁₃H₁₀N₄O₃S.0,25H₂O.0,25C₂H₅OH (C,H,N).

### EXEMPLE 26:

### Préparation de la 5-méthyl-3-nitro-4-(benzoxazol-2-yl)thio-pyridin-2(1H)-one (composé 9d).

Comme décrit pour le composé **7a** à l'exemple 10, un mélange du composé **6a** (0,50 g, 2,6 mmoles) dans 20 ml d'éthanol et 0,55 ml de triéthylamine (4,0 mmoles) est agité jusqu'à homogénéité. On ajoute du 2-mercaptobenzoxazole (0,40 g, 2,6 mmoles) et on agite le mélange à reflux pendant 3 h et à la température ambiante pendant 15 h. On évapore le solvant et on cristallise le résidu dans l'éthanol pour donner le produit 9**d** (0,22 g, 27%) sous forme d'un solide brun: point de fusion 180-181°C; RMN-¹H (DMSO-*d*₆) δ 8,91 (1H, large, NH-1), 7,85 (1H, s, H-6), 7,81-7,70 (2H, m, H-5' et 8'), 7,50-7,39 (2H, m, H-6' et 7'), 2,10 (3H, s, CH₃-5). Anal. C₁₃H₉N₃O₄S.0,5C₂H₅OH (C,H,N,S).

### EXEMPLE 27:

### Préparation de la 5-méthyl-3-nitro-4-(benzothiazol-2-yl)thio-pyridin-2(1H)-one (composé 9e).

Comme décrit pour le composé **7a** à l'exemple 10, un mélange du composé **6a** (0,10 g, 0,5 mmole) dans 10 ml d'éthanol et 10 ml de triéthylamine est agité jusqu'à homogénéité. On ajoute du 2-mercaptobenzothiazole (0,09 g, 0,5 mmole) et on agite le mélange à la température ambiante pendant 15 h et à reflux pendant 4 h. On évapore toutes les substances volatiles et on ajoute 10 ml d'eau. On agite la suspension à la température ambiante pendant 1 h. Le solide jaune est isolé par filtration, lavé avec de l'eau et séché sur P₂O₅ sous vide pour donner le produit 9**e** (0,10 g, 60%) sous forme de cristaux jaunes: point de fusion 240°C; RMN-¹H (DMSO-*d*₆) δ 12,90 (1H, large, NH-1), 8,09 (1H, dd, J₁ = 7Hz, J₂ = 1Hz, H-8'#), 7,96 (1H, dd, J₁ = 7Hz, J₂ = 1Hz, H-5'#), 7,88 (1H, s, H-6), 7,56 (1H, td, J₁ = 7Hz, J₂ = 1Hz, H-7'*), 7,47 (1H, td, J₁ = 7Hz, J₂ = 1Hz, H-6'*), 2,10 (3H, s, CH₃-5). Anal. C₁₃H₉N₃O₃S₂ (C,H,N).

### EXEMPLE 28:

### Préparation de la 5-méthyl-3-nitro-4-(thiazolin-2-yl)thio-pyridin-2(1H)-one (composé 9f).

Comme décrit pour le composé **7a** à l'exemple 10, un mélange du composé **6a** (0,50 g, 2,6 mmoles) dans 10 ml d'éthanol et 20 ml de triéthylamine est agité jusqu'à homogénéité. On ajoute de la 2-mercaptothiazoline (0,32 g, 2,6 mmoles) et on agite le mélange à reflux pendant 8 h et à la température ambiante pendant 15 h. On évapore toutes les substances volatiles et on ajoute 20 ml d'eau. On agite la suspension à la température ambiante pendant 48 h. Le solide est isolé par filtration et lavé avec de l'eau. On le purifie par chromatographie sur colonne en utilisant comme éluant un mélange dichlorométhane/éthanol (1:0 à 9:1) pour donner le produit 9**f** (0,05 g, 7%) sous forme de cristaux bruns: point de fusion 177°C; RMN-¹H (DMSO-*d*₆) δ 13,10 (1H, large, NH-1), 7,41 (1H, s, H-6), 4,21 (2H, t, J =8 Hz, H-4'), 3,45 (2H, t, J = 8Hz, H-5'),2,20 (3H, s, CH₃-5). Anal. C₉H₉N₃O₃S₂.0,25C₂H₅OH (C,H,N).

### EXEMPLE 29:

### Préparation de la 5-méthyl-3-nitro-4-(N-méthylimidazol-2-yl)thio-pyridin-2(1H)-one (composé 9g).

Comme décrit pour le composé **7a** à l'exemple 10, un mélange du composé **6a** (0,50 g, 2,6 mmoles) dans 20 ml d'éthanol et 0,55 ml de triéthylamine (4,0 mmoles) est agité jusqu'à homogénéité. On ajoute du 2-mercapto-1-méthylimidazole (0,30 g, 2,6 mmoles) et on agite le mélange à reflux pendant 6 h et à la température ambiante pendant 15 h. On évapore le solvant et on ajoute 15 ml d'eau. On agite la suspension à la température ambiante pendant 1 h. Le solide est isolé par filtration, lavé avec de l'eau et séché sur P₂O₅ sous vide pour donner le produit 9**g** (0,60 g, 85%) sous forme de cristaux jaunes: point de fusion 260-265°C; RMN-1H (DMSO-*d*₆) δ 9,45 (1H, s, NH-1), 7,51 (1H, s, H-6), 7,38 (1H, t, J = 1Hz, H-5'*), 7,04 (1H, d, J = 1Hz, H-4'*), 3,61 (3H, s, N-CH₃), 1,82 (3H, s, CH₃-5). Anal. C₁₀H₁₀N₄O₃S (C,H,N,S).

### EXEMPLE 30:

### Préparation de la 5-méthyl-3-nitro-4-(2-pyridyl)thio-pyridin-2(1H)-one (composé 9h).

Comme décrit pour le composé **7a** à l'exemple 10, un mélange du composé **6a** (0,10 g, 0,5 mmole) dans 10 ml d'éthanol et 10 ml de triéthylamine est agité jusqu'à homogénéité. On ajoute de la 2-mercaptopyridine (0,06 g, 0,5 mmole) et on agite le mélange à la température ambiante pendant 48 h. On évapore le solvant et on ajoute 10 ml d'eau. On agite la suspension à la température ambiante pendant 3 h. Le solide est isolé par filtration, lavé avec de l'eau et séché sur P₂O₅ sous vide pour donner le produit 9**h** (0,09 g, 66%) sous forme de cristaux bruns: point de fusion 195-196°C; RMN-¹H (DMSO-*d*₆) δ 12,89 (1H,large,NH-1), 8,47-8,44 (1H, m, H-6'), 7,85-7,76 (1H, m, H-4'), 7,73 (1H, s, H-6), 7,41-7,28 (2H, m, H-3' et 5'), 1,93 (3H, s, CH₃-5). Anal. C₁₁H₉N₃O₃S (C,H,N,S).

### EXEMPLE 31:

### Préparation de la 3-amino-5-méthyl-4(benzoxazol-2-yl)thio-pyridin-2(1H)-one (composé 10).

Comme décrit pour le composé **8a** à l'exemple 17, on ajoute du chlorure stanneux dihydraté (420 mg, 1,9 mmoles) à une suspension de **composé 9d** (110 mg, 0,4 mmole) dans l'acétate d'éthyle (10 ml). On chauffe le mélange à reflux pendant 6 h. Après refroidissement à 0°C, on ajoute 10 ml d'eau glacée et on basifie la solution avec une solution saturée de carbonate de sodium. On sépare les deux phases et on extrait la phase aqueuse avec 3 x 20 ml d'acétate d'éthyle. Les phases organiques rassemblées sont lavées avec une solution aqueuse saturée de chlorure de sodium (3 x 10 ml), séchées sur du sulfate de magnésium et concentrées. On lave le résidu avec du dichlorométhane pour donner le produit **10** (10 mg, 10%) sous forme d'un solide brun: point de fusion 296-297°C; RMN-¹H (DMSO-*d*₆) δ 11,36 (1H, s large, NH-1), 9,98 (1H, s, NH₂-3), 9,83 (1H, s, NH₂-3), 8,35 (1H, d, J = 7Hz, H-5'*), 6,99 (1H, s, H-6), 6,94-6,82 (3H, m, H-6',7' et 8'*), 2,16 (3H, s, CH₃-5). Anal. C₁₃H₁₁N₃O₂S.H₂O(C,H,N).

### EXEMPLE 32:

### Préparation de la 3-formamido-5-méthyl-4-(3',5'-diméthylphényl)thio-pyridin-2(1H)-one (composé 11a).

On ajoute de l'acide formique (2 ml) à une solution de l'amine **8a** (0,10 g, 0,4 mmole) dans du formiate d'éthyle (distillé auparavant sur de l'hydrure de calcium) (8 ml). On chauffe le mélange à reflux pendant 12 h. Après évaporation des substances volatiles, on lave le résidu deux fois avec de l'éthanol et une fois avec de l'acétate d'éthyle. On le purifie par chromatographie sur colonne en utilisant comme éluant un mélange dichlorométhane/éthanol (95:5) et à la fin, de l'acétate d'éthyle, pour donner **le composé 11a** (0,06 g, 58%) sous forme d'une poudre blanche: point de fusion 221-222°C; RMN-¹H (DMSO-*d*₆) δ 11,96 (1H, s, NH-1), 8,28 (1H, s, NH-3), 7,23 (1H, s, H-6), 6,88 (1H, s, H-4'), 6,78 (2H, s, H-2' et 6'), 2,23 (6H, s, CH₃-3' et 5'), 1,85 (3H, s, CH₃-5). Anal. C₁₅H₁₆N₂O₂S (C,H,N,S).

### EXEMPLE 33:

### Préparation de la 3-acétamido-5-méthyl-4-(3',5'-diméthylphényl)thio-pyridin-2(1H)-one (composé 11b).

On chauffe à reflux pendant 1 h 30 une solution de l'amine **8a** (0,10 g, 0,4 mmole) et d'anhydride acétique (0,05 ml, 0,39 mmole) dans l'acide acétique (20 ml). Après évaporation des solvants, on ajoute 10 ml d'eau et on neutralise le mélange à 0°C avec une solution aqueuse diluée d'ammoniac. Après filtration, on lave le résidu avec du cyclohexane (2 x 5 ml). On obtient le produit **11b** (0,10 g, 86%) sous forme d'un solide beige clair: point de fusion 138°C; RMN-¹H (DMSO-*d*₆) δ 11,84 (1H, s, NH-1), 9,41 (1H, s, NH-3), 7,20 (1H, s, H-6), 6,87 (1H, s, H-4'), 6,81 (2H, s, H-2' et 6'), 2,22 (6H, s, CH₃-3' et 5'), 1,99 (3H, s, CH₃-5), 1,81 (3H, s, CH₃CO). Anal. C₁₆H₁₈N₂O₂S.H₂O (C,H,N,S).

### EXEMPLE 34:

### Préparation de la 5-méthyl-3-propionamido-4-(3',5'-diméthylphényl)thio-pyridin-2(1H)-one (composé 11c).

On ajoute à une solution de l'amine **8a** (0,10 g, 0,40 mmole) et de triéthylamine (0,04 ml, 0,38 mmole) dans du dichlorométhane (5 ml), à 0°C, du chlorure de propionyle fraîchement distillé (0,04 ml, 0,41 mmole) (le récipient est équipé d'un piège contenant du CaCl₂). On agite le mélange à la température ambiante pendant 5 h. On évapore le solvant, on ajoute de l'eau et on isole le solide par filtration. On purifie le résidu par chromatographie sur colonne en utilisant comme éluant un mélange dichlorométhane/éthanol (94:6) et à la fin, de l'acétate d'éthyle pour obtenir le composé **11c** (0,10 g, 90%) sous forme d'une poudre blanche: point de fusion 186-188°C; RMN-¹H (DMSO-*d*₆) δ 11,86 (1H, s, NH-1), 8,60 (1H, s, NH-3), 7,21 (1H, s, H-6), 6,88 (1H, s, H-4'), 6,80 (2H, s, H-2' et 6'), 4,04 (2H, q, J = 7Hz, CO*CH*_{*2*}CH₃), 2,22 (6H, s, CH₃-3' et 5'), 1,82 (3H, s, CH₃-5), 1,20 (3H, t, J = 7Hz, COCH₂*CH*_{*3*}). Anal. C₁₇H₂₀N₂O₂S.1,25H₂O (C,H,N,S).

### EXEMPLE 35:

### Préparation de la 3-heptanamido-5-méthyl-4-(3',5'-diméthylphényl)thio-pyridin-2(1H)-one (composé 11d).

On chauffe une solution de l'amine **8a** (100 mg, 0,38 mmole) et d'anhydride heptanoïque (0,14 g, 0,58 mmole) dans du toluène (4 ml) à 100°C pendant 1 h 30. Après évaporation du toluène, on ajoute 5 ml d'éther diéthylique. Une fois le précipité obtenu, le solvant est éliminé à l'aide d'une pipette. On lave deux fois le solide de cette manière avec de l'éther diéthylique. Après filtration, on recristallise le résidu dans l'acétate d'éthyle. On obtient le produit **11d** (17 mg, 50%) sous forme de microcristaux jaunes: point de fusion 212-213°C; RMN-¹H (DMSO-*d*₆) δ 11,86 (1H, s, NH-1), 9,34 (1H, s, NH-3), 7,19 (1H, s, H-6), 6,86 (1H, s, H-4'), 6,76 (2H, s, H-2' et 6'), 2,28 (2H, t, J = 7Hz, CO*CH*_{*2*}), 2,22 (6H, s, CH₃-3' et 5'), 1,81 (3H, s, CH₃-5), 1,53 (2H, dd, J = 7 Hz, COCH₂*CH*_{*2*}), 1,28-1,21 (6H, m, (CH₂)3), 0,86 (3H, t, J = 6,5 Hz, (CH₂)₅*CH*_{*3*}). Anal. C₂₁H₂₈N₂O₂S (C,H,N,S).

### EXEMPLE 36:

### Préparation de la 5-méthyl-3-phénylacétamido-4-(3',5'-diméthylphényl)thio-pyridin-2(1H)-one (composé 11e).

On ajoute un équivalent molaire de triéthylamine (0,035 ml, 0,25 mmole) à une suspension de l'amine **8a** (65 mg, 0,25 mmole) dans du dichlorométhane (5 ml). On ajoute goutte à goutte à ce mélange refroidi dans l'eau glacée, une solution distillée de chlorure de phénylacétyle (39 mg, 0,25 mmole) dans du dichlorométhane (1 ml). Le mélange est agité à la température ambiante pendant 2 h. Après évaporation du solvant, on ajoute 10 ml d'eau. Après filtration, on purifie le résidu par chromatographie sur colonne en utilisant comme éluant un mélange dichlorométhane/éthanol (95:5). On obtient le composé **11e** (53 mg, 56%) sous forme de cristaux verts: point de fusion 200-202°C; RMN-¹H (DMSO-*d*₆) δ 11,88 (1H, s, NH-1), 9,67 (1H, s, NH-3), 7,37-7,21 (5H, m, C₆H₅), 6,87 (1H, s, H-4'), 6,76 (2H, s, H-2' et 6'), 3,67 (2H, s, CH₂), 2,22 (6H, s, CH₃-3' et 5'), 1,80 (3H, s, CH₃-5). Anal. C₂₂H₂₂N₂O₂S (C,H,N).

### EXEMPLE 37:

### Préparation de la 5-méthyl-3-N-éthoxycarbamoyl-4-(3',5'-diméthylphényl)thio-pyridin-2(1H)-one (composé 11f).

On ajoute de la triéthylamine (0,10 g, 0,50 mmole) à une solution de l'amine **8a** (0,05 g, 0,20 mmole) dans l'éthanol (2 ml). On ajoute goutte à goutte à ce mélange refroidi dans l'eau glacée, du chloroformiate d'éthyle fraîchement distillé (0,65 g, 6,00 mmoles). Le mélange est agité à la température ambiante pendant 48 h. Après évaporation du solvant, on ajoute 5 ml d'eau. Après filtration, on purifie le solide rouge par chromatographie sur colonne en utilisant comme éluant un mélange dichlorométhane/éthanol (98:2) pour donner l'amine récupérée **8a** (0,005 g, 10%) et le composé **11f** (0,01 g, 26%) sous forme d'un solide blanc: point de fusion 208-210°C; RMN-¹H (DMSO-*d*₆) δ 11,83 (1H, s, NH-1), 8,60 (1H, s, NH-3), 7,21 (1H, s, H-6), 6,88 (1H, s, H-4'), 6,80 (2H, s, H-2' et 6'), 4,04 (2H, q, J=7Hz, O*CH*_{*2*}CH₃), 2,22 (6H, s, CH₃-3' et 5'), 1,82 (3H, s, CH₃-5), 1,19 (3H, t, J=7Hz, OCH₂*CH*_{*3*}) Anal. C₁₇H₂₀N₂O₃S (C,H,N,S).

### EXEMPLE 38:

### Préparation de la 3-acétamido-5-éthyl-6-méthyl-4-(3',5'-diméthylphényl)thio-pyridin-2(1H)-one (composé 11g).

On chauffe à reflux pendant 5h30 une solution de l'amine **8c** (0,20 g, 0,7 mmole) et d'anhydride acétique (0,09 ml, 0,9 mmole) dans l'acide acétique (40 ml). Après évaporation des solvants, on ajoute 20 ml d'eau glacée et on neutralise le mélange à 0°C avec une solution aqueuse diluée d'ammoniac. Le mélange est agité à température ambiante pendant une nuit. Après filtration, on lave le résidu avec du cyclohexane (2 x 10 ml). On obtient le produit **11g** (0,17 g, 74%) sous forme d'un solide brun clair: point de fusion 238-239°C; RMN-¹H (DMSO-*d*₆) δ 11,90 (1H, s large, NH-1), 9,18 (1H, s large, NH-3), 6,83 (1H, s, H-4'), 6,77 (2H, s, H-2' et 6'), 2,22 (9H, s, CH₃-5, 3' et 5'), 1,87 (3H, s, CH₃CO), 0,85 (3H, t, J=7Hz, *CH*_{*3*}CH₂). Le signal de CH₃*CH*_{*2*} et le signal du DMSO se chevauchent. Anal. C₁₈H₂₂N₂O₂S.0,45H₂O (C,H,N,S).

### EXEMPLE 39:

### Préparation de la 4-chloro-5-éthyl-6-méthyl-3-carbéthoxypyridin-2(1H)-one (composé 14).

Comme pour le procédé décrit pour l'obtention du composé **6a** décrit par Nguyen et al. (1992, précédemment cités), on ajoute de l'oxychlorure de phosphore (5,4 ml, 56,8 mmoles) à une solution du composé 13 (3,00 g, 13,3 mmoles) et de chlorure de benzyltriéthylammonium (12,12 g, 53,2 mmoles) dans de l'acétonitrile (60 ml). Le mélange obtenu est chauffé à reflux pendant 6 h pour donner une solution rouge. Après évaporation du solvant, on ajoute 20 ml d'eau glacée et on agite le mélange à 0°C pendant 4 h. Le précipité est recueilli, lavé avec du cyclohexane (2 x 4 ml) et cristallisé dans l'acétate d'éthyle pour donner **le composé 14** (2,3 g, 71 %) sous forme de cristaux blancs: point de fusion 167°C; RMN-¹H (DMSO-*d*₆) δ 12,24 (1H, s, NH-1), 4,28 (2H, q, j=7Hz, COO*CH*_{*2*}CH₃), 2,31 (3H, s, CH₃-6), 1,29 (3H, t, j=7Hz, COOCH₂*CH*_{*3*}), 1,06 (3H, t, j=7Hz, CH₂*CH*_{*3*}), le signal de *CH*_{*2*}CH₃ et le signal de DMSO se chevauchent. Anal. C₁₁H₁₄NO₃Cl (C,H,N,CI).

### EXEMPLE 40:

### Préparation de la 5-éthyl-6-méthyl-3-carbéthoxy-4-(3',5'-diméthylphényl)thio-pyridin-2(1H)-one (composé 15).

Comme pour le procédé décrit pour l'obtention du composé **7a** à l'exemple 10, un mélange du composé **15** (1,00 g, 4,1 mmoles) dans 10 ml d'éthanol et 1 ml de triéthylamine est agité jusqu'à homogénéité. On ajoute du 3,5-diméthylthiophénol (0,61 ml, 4,5 mmoles). Après 12 h à reflux, on isole le précipité blanc par filtration et on le cristallise dans l'acétate d'éthyle. On obtient le produit **15** (1,05 g, 82%) sous forme de cristaux blancs: point de fusion 202°C; RMN-¹H (DMSO-*d*₆) δ 12,17 (1H, s, NH-1), 7,22 (1H, s, H-4'), 6,92 (1H, s, H-2' et 6'), 4,09 (2H, q, J=7Hz, COO*CH*_{*2*}CH₃), 2,45 (2H, q, J=7Hz, *CH*_{*2*}CH₃), 2,27 (3H, s, CH₃-6), 2,25 (6H, s, CH₃-3' et 5'), 1,15 (3H, t, J=7Hz, COOCH₂*CH*_{*3*}), 0,85 (3H, t, J=7Hz,CH₂*CH*_{*3*}). Anal. C₁₉H₂₃NO₃S (C,H,N,S).

### EXEMPLE 41:

### Préparation de la 5-éthyl-6-méthyl-4-(3',5'-diméthylphényl)thio-pyridin-2(1H)-one (composé 16).

On dissout le composé **15** (120 mg, 0,34 mmole) dans 6 ml de mélange tétrahydrofurane/H₂O/HCl à 37% (18:3:4).On agite le mélange à 75°C pendant 10 jours. On évapore le tétrahydrofurane et on ajoute 5 ml d'eau. On agite le mélange et on élimine l'eau. On cristallise le résidu dans l'éthanol (25 ml) pour donner le produit **16** (50 mg, 59%) sous forme de paillettes incolores: point de fusion 277-278°C; RMN-¹H (DMSO-*d*₆) δ 11,26 (1H, s, NH-1), 7,22 (3H, s, H-2', 4' et 6'), 5,25 (1H, s, H-3), 2,36 (6H, s, CH₃-3' et 5'), 2,21 (3H, s, CH₃-6), 1,13 (3H, t, J= 7,5 Hz, CH₂*CH*_{*3*}). Le signal de *CH*_{*2*}CH₃ et le signal du DMSO se chevauchent. Anal. C₁₆H₁₉NOS.0,25H₂O (C,H,N,S)

### EXEMPLE 42:

### Activité biologique des composés selon l'invention.

### 1°) Matériel et méthodes.

### Evaluation de l'activité antivirale des composés

On a évalué les effets des composés sur la réplication du VIH-1 (voir tableau 2), sur des cellules CEM-SS (une lignée cellulaire du lignage lymphocytique) fortement infectées par VIH-1 LAI comme décrit par Moog et al. (Antiviral Research (1994), 24, 275-288). Les CEM-SS et le VIH-1 résistant à la Névirapine (N119) portant une mutation ponctuelle au niveau du codon 181 de la TI, ont été fournis respectivement par P. NARA et D. RICHMAN par l'intermédiaire du AIDS Research and Reference Reagent Program, Division of AIDS, NIAID, NIH (USA) (Références catalogue : N°776 et n° 1392).

On a mesuré la production de virus en quantifiant l'activité de transcriptase inverse associée aux particules virales libérées dans le surnageant de culture. Brièvement, on a infecté les cellules avec 100 TCID₅₀ pendant 30 mn; après adsorption du virus, les particules non liées ont été éliminées par deux lavages et les cellules ont été cultivées en présence de différentes concentrations des composés testés pendant 5 jours avant la détermination de la production virale. La concentration inhibitrice à 50% de la multiplication virale (IC₅₀) provient du tracé informatisé de l'effet médian des résultats de dose-effet (Chou et al. Elsevier Science Publishers, Cambridge, U.K. (1985), 19-28).

Dans des expériences parallèles, on a mesuré la cytotoxicité des molécules sur des cellules non infectées, après une incubation de 5 jours en présence de ces molécules, en utilisant un dosage colorimétrique (épreuve au MTT) basé sur la capacité des déshydrogénases mitochondriales des cellules vivantes à réduire le bromure de 3-(4,5-diméthylthiazol-2-yl)-2,5-diphényltétrazolium en formazan (Mosmann, et al. J. Immunol. Methods (1983), 65, 55-63). La concentration cytotoxique à 50% (CC₅₀) est la concentration à laquelle la DO₅₄₀ a été réduite de moitié et est calculée en utilisant le programme mentionné ci-dessus.

### Expression et purification de l'enzyme TI du VIH-1 recombinant

On a utilisé des cellules de levure, transformées avec le vecteur pAB 24/TI-4 pour purifier l'enzyme TI du VIH-1 recombinante comme décrit par Sallafranque-Andreola et al.(1989 Eur. J. Biochem. 184,364-374).

Le système pour l'expression de la TI du VIH-2 dans *E.coli (B.* Müller et al. (1991 J. Biol. Chem. 266, 14709-14713) a été offert par Dr. R. Goody. La TI du VIH-2 a été purifiée comme la TI du VIH-1.

### Dosages de transcriptase inverse

On a effectué l'incubation à 37°C pendant 10 mn en présence de différentes matrices-amorces.
a) Poly C-oligo dG: le mélange réactionnel contenait un volume final de 0,05 ml, Tris-HCI 50 mM pH 8,0, MgCl₂ 5 mM, dithiothreitol 4 mM, 0,48 A₂₆₀/ml de poly C-oligo dG (5:1), 1,0 µCi de [³H]dGTP (28 Ci/mmole), dGTP 2 µM, KCI 80 mM, 1 µg de sérum-albumine bovine et Tl 20-50 nM.
b) Poly A-oligo dT: les mêmes conditions que dans a), sauf que l'on a utilisé 0,48 A₂₆₀/ml de poly A-oligo dT (5:1), 0,5 µCi de [³H]dTTP (46 Ci/mmole), dTTP 20 µM.

On a arrêté les réactions en ajoutant à froid 1 ml d'acide trichloroacétique à 10% plus du pyrophosphate de sodium 0,1 M. Les précipités ont été filtrés à travers des membranes de nitrocellulose, lavés avec de l'acide trichloroacétique à 2%, séchés et comptés dans un mélange de scintillation PPO/POPOP/toluène.

### Transcription inverse

Le plasmide pmCG6 contenant le fragment nucléotidique 1-4005 du VIH-1 (pmal) dans psP64, sous le contrôle du promoteur du bactériophage T7 a été offert par Dr. J.L. Darlix. On a utilisé une souche de *E. coli* HB 101 (1035) recA- pour l'amplification du plasmide. Après digestion de ce clone avec Hinc II et transcription in vitro en utilisant de l'ARN polymérase de T7, on a obtenu des ARN à partir de la position +50 de la séquence pmal. On a effectué la transcription in vitro et la transcription inverse de la manière décrite par B. Bordier et al. (1992 Nucleic Acids Res. 20, 5999-6006).

### Expériences d'inhibition

On a dissous tous les composés dans du diméthylsulfoxyde (DMSO). On a préparé les témoins en présence de la même concentration finale de DMSO. IC₅₀ est la concentration nécessaire pour inhiber l'activité de la TI du VIH-1 recombinante à 50%.

### 2°) Résultats

### Inhibition de la multiplication du VIH-1

On a étudié trente deux composés selon l'invention pour leur activité biologique anti-VIH-1. Plusieurs molécules ont présenté des propriétés antivirales importantes (voir Tableau 2). Parmi les meilleurs inhibiteurs présentant un indice de sélectivité supérieur à 20, plusieurs ont été testés sur une souche résistante à la Névirapine (voir Tableau 3). Il s'est avéré que le composé **7c** conserve une bonne activité anti-VIH-1 avec une IC₅₀ de 260 nM sur cette souche résistante. Ce composé a alors été testé vis-à-vis d'autres souches et d'autres lignées cellulaires (Tableau 4).

### Inhibition de la transcriptase inverse

On a testé les composés qui se sont révélés actifs contre le VIH-1 en culture cellulaire, sur une TI de VIH-1 recombinante. La concentration inhibant 50% de l'activité de TI (IC₅₀) pour chaque composé est donnée dans le Tableau 5. Les composés **7c** et **8c** sont les meilleurs inhibiteurs avec des IC₅₀, respectivement de 30 nM et 15 nM. Un dérivé de HEPT, la 1-benzyloxyméthyl-6-(phénylthio)thymine ou BPT (Baba et al.,Proc. Natl. Acad. Sci. USA, 1991, 88, 2356-2360) testé dans les mêmes conditions et utilisé comme composé de référence d'inhibiteurs non nucléosidiques a donné une valeur d'IC₅₀ de 400 nM.

Des études complémentaires ont été entreprises pour élucider la nature de l'inhibition. Ces études ont été effectuées principalement avec les composés **7c** et **8c**.

On a déterminé l'inhibition de TI en utilisant des couples matrices-amorces différents. Il est connu que les inhibiteurs non nucléosidiques de TI présentent différents niveaux d'inhibition selon la matrice-amorce utilisée pour mesurer l'activité de TI (De Clercq et al. (1993) Médicinal Research Reviews, 13, 229-258).

Les courbes de dose-réponse pour le composé **7c** en présence de deux matrices-amorces différentes sont représentées à la figure 9. On a obtenu une meilleure inhibition en présence de poly C-oligo dG par rapport à poly A-oligo dT. Le composé **8c** a donné le même résultat. On a également obtenu une forte inhibition en utilisant l'ARN du VIH-1 comme matrice naturelle (Figure 10).

Tous les inhibiteurs non nucléosides identifiés jusqu'à présent sont spécifiques du VIH-1 et n'inhibent pas la TI du VIH-2. On a testé les composés **7c** et **8c** avec les deux Tl. Comme le montre la figure 9, à des concentrations où la TI du VIH-1 est bien inhibée, l'activité de la Tl du VIH-2 n'est pas affectée. Ce comportement discriminatoire envers la TI du VIH-1 par rapport à la TI du VIH-2 est une propriété courante des inhibiteurs non nucléosidiques.

Des analyses de cinétique enzymatique montrent que l'inhibition de la TI du VIH-1 par les composés **7c** (ou **8c**) est non compétitive en ce qui concerne le substrat dGTP en présence du couple matrice-amorce poly C-oligo dG (Figure 12). Lorsqu'on utilise poly A-oligo dT comme couple matrice-amorce, on obtient une inhibition de type compétitif (Figure 13).

### EXEMPLE 43:

### Etude de la perméabilité du virion isolé aux inhibiteurs de la transcriptase inverse du VIH-1.

### 1. Matériels et Méthodes

### 1.1 Préparation des surnageants viraux:

Des cellules H9 chroniquement infectées par HIV-1LAI (10⁶/ml) sont cocultivées avec des cellules MT4(10⁶/ml) pendant 48 heures en milieu RMPI supplémenté par 10% de Sérum Foetal de Veau (SVF). Les cellules sont alors éliminées par centrifugation puis le surnageant est filtré (0,45µm) afin d'éliminer les cellules résiduelles et les débris cellulaires.

### 1.2 Séparation des particules virales:

Après avoir été incubés avec les divers inhibiteurs, les surnageants viraux (200 µl) sont déposés dans la partie filtrante d'un tube VectaSpin (WHATMAN) muni d'une membrane ANOPORE de porosité 0,02µm. Ils sont soumis pendant 10 minutes à une centrifugation dont l'accélération atteint 6000g au niveau de la membrane filtrante. Les culots viraux ainsi obtenus (10-15 µl) sont lavés 3 fois dans les mêmes conditions avec 500 µl de RPMI puis ramenés à leurs volumes initiaux grâce à du RPMI contenant 10% de SVF.

### 1.3. Culture cellulaire:

Les cellules HT4LacZ-1 sont maintenues en milieu DMEM (glucose 4,5 g/l)supplémenté par 10% de sérum de veau foetal en présence de Gentamycine (50 mg/l). Ces cellules sont issues de cellules Hela dans lesquelles ont été clonés, d'une part le gène codant pour le CD4 humain afin de les rendre susceptibles à l'infection par le VIH (Chesebro et Wehrly, 1988, J. Virol., 62, 3779-3788), d'autre part le gène de la β-Galactosidase qui, placé sous la dépendante du LTR du VIH-1 sert de marqueur à l'infection virale (Rocancourt et al. (1990) J. Virol. 1990 64-6, 2660-2668). Lorsque le virus infecte de telles cellules après son intégration dans le génome cellulaire, la production de la protéine régulatrice TAT active le LTR du VIH. Il en résulte une activation du gène de la β-Galactosidase qui conduit à sa production dans le milieu intracellulaire et plus particulièrement dans le noyau. C'est cette protéine qui sert de marqueur de l'infection.

### 1.4 Mesure de l'infectivité:

Le soir du jour 0, les cellules HT4LacZ-1 sont diluées à raison de 75000 cellules par ml de milieu. 100 µl de cette suspension sont alors répartis en plaque à fond plat de 96 puits et cultivés la nuit à 37°C (5% de CO₂).

Le matin du jour 1, le surnageant est éliminé par aspiration. Chaque puits est alors complété par 200 µl des dilutions des surnageants viraux à tester. Chaque dilution est réalisée en milieu DMEM (glucose 4.5 g/l) supplémenté par 10% de SVF. Les plaques sont alors placées à 37°C en présence de 5% de CO₂.

Après 48 heures de culture, le surnageant est aspiré, puis chaque puits est lavé 3 fois en PBS avant d'être complété par 200 µl de tampon de révélation (Tris-HCl 50mM pH=8,5. ONPG 5 mg/ml β-Mercapto-éthanol 7µl/ml Triton X100 0,05%). Après 4 heures d'incubation à 37°C, la densité optique de chaque puits est mesurée.

### 2. Résultats.

### 2.1 Validation de l'ultrafiltration virale

Le protocole décrit plus haut a été appliqué à un surnageant viral issu d'une coculture H9-MT4. Après chaque centrifugation, le culot viral est repris dans son volume initial à l'aide de milieu RMPI 16/40 contenant 10% de sérum de veau foetal, puis un aliquote est prélevé aux fins d'analyses (le volume initial des centrifugations successives étant modifié, le volume de lavage est réajusté à chaque étape). Trois concentrats viraux ainsi que trois filtrats sont ainsi obtenus. Ceux-ci ont été analysés d'une part par mesure de l'activité transcriptase inverse (reflet de la quantité de virus présent), d'autre part par mesure de leur pouvoir infectieux (dépendant directement de l'intégrité de chaque particule).

### 2.1.1. Mesure de l'activité transcriptase inverse (TI).

Chacun des concentrats et filtrats viraux réajustés à leur volume initial est analysé quant à son activité Tl. Pour cela, 50 µl de surnageant sont testés en présence d'un duplex matrice/amorce constitué de poly rA et d'oligo dT en présence de dTTP-H³. Les résultats sont exprimés sur la figure 14.

Les mesures de l'activité Ti des trois concentrats révèlent une perte d'activité de l'ordre de 5% après trois filtrations. Les valeurs observées dans les trois filtrats se confondent au bruit de fond, indiquant l'excellente homogénéité des membranes ANOPORE.

### 2.1.2. Mesure de l'infectiosité

Afin d'évaluer la toxicité éventuelle du processus sur la particule virale, des mesures d'infectivité ont été réalisées. Pour cela, différentes dilutions des concentrats viraux sont mis en contact avec des cellules MT2 dans 200 µl de milieu RMPI supplémenté par 10% de sérum de veau foetal. Après 3 heures d'incubation, les cellules sont lavées deux fois puis remises en culture pendant 96 heures. A ce terme, le surnageant est prélevé , les cellules éliminées par centrifugation, puis le surnageant est testé quant à son activité transcriptase inverse. Les résultats représentés dans la figure 15 montrent que le pouvoir infectieux n'est pas significativement affecté par les étapes d'ultrafiltration.

L'ultrafiltration sur membrane ANOPORE 0,02µ permet donc une séparation rapide, efficace et non traumatisante des particules virales.

### 2.2 Activité des inhibiteurs de la Ti du VIH-1 sur le vision isolé.

La concentration de chacun des inhibiteurs de la TI du VIH-1 est ajustée à 50 mM en DMSO. Les dilutions ultérieures sont réalisées en RPMI 1640. 2 µl des dilutions adéquates de chacun de ces inhibiteurs sont ajoutés à 198 µl d'une suspension virale fraîchement décongelée. Après 3 heures d'incubation à température ambiante, ces suspensions sont filtrées sur tubes VecraSpin 0,02µ puis lavées 3 fois avec 500 µl de RPM1 1640. Les culots viraux sont alors ajustés à 200 µL avec du milieu de culture (RPMI - 10% SVF), puis testées quant à leur infectivité sur cellules HT4 LacZ.

Les résultats présentés dans le tableau 6 montrent clairement qu'à l'exclusion du composé 7c aucun des inhibiteurs ne s'est montré actif sur le virion isolé. Afin de vérifier que ces effets n'étaient pas dus à d'éventuelles dégradations, chacun de ces inhibiteurs a été simultanément évalué sur cellules HT4LacZ en présence de virus. Tous ont montré des activités comparables à celles décrites dans la littérature. Les résultats obtenus pour la Nevirapine à 10 et 1 µM, dans l'état actuel de l'expérimentation sont probablement liés aux fluctuations expérimentales. Il faut en outre ajouter que des inhibitions significatives de l'infectivité ont été observées avec le TIBO R82913 à la concentration de 100 µM. Quoiqu'il en soit, seul le composé 7c s'est révélé véritablement actif sur le virion isolé, à des concentrations descendant jusqu'à 100 nM.

### 2.3 Inhibition de la Ti sur le virion isolé

Afin de vérifier que l'inhibition de l'infectivité était bien due à l'activité anti-transcriptase inverse du composé 7c, nous avons mesuré l'activité Ti sur une matrice amorce poly rA/oligo dT après que les virions isolés aient été incubés 3 heures avec ce composé.

Le composé 7c semble donc pénétrer l'enveloppe et la capside virale pour se fixer sur la TI et en bloquer l'activité.

D'autre part des expériences préliminaires sur la cinétique de pénétration du composé 7c indiquent que des incubations de 45 minutes suffisent à induire des inhibitions d'infectivité supérieures à 98%.

### 3. Conclusion

Les présents résultats montrent que les principaux inhibiteurs utilisés en clinique humaine sont totalement inactifs sur le virion isolé (AZT, d4T, 3TC, ddl. TIBO R82913, HEPT NEVIRAPINE). Le composé de formule 7c s'est montré capable, avoir après pénétré la particule virale, d'inhiber la transcriptase inverse et de supprimer l'infectivité virale.

Ce composé se comporte donc comme un composé parfaitement original quant à sa capacité de pénétration dans le virion isolé.

**TABLEAU 1:**

| **Analyses élémentaires des composés selon l'invention** | | | | | | | | | | |
|---|---|---|---|---|---|---|---|---|---|---|
| **Composé** | **% Calculé** | | | | | **% mesuré** | | | | |
| | C | H | N | S | Cl | C | H | N | S | Cl |
| 4c | 62.74 | 7.19 | 9.15 | | | 62.62 | 7.23 | 9.15 | | |
| 5b | 44.57 | 4.54 | 14.85 | | | 44.83 | 4.25 | 14.69 | | |
| 5c | 48.48 | 5.09 | 14.14 | 32.29 | | 48.89 | 4.67 | 14.29 | | |
| 5g | 52.44 | 2.93 | 13.59 | | | 52.45 | 2.94 | 13.56 | | |
| 7a | 57.04 | 4.92 | 9.50 | 10.85 | | 57.22 | 5.10 | 9.57 | 10.60 | |
| 7b | 59.17 | 5.26 | 9.20 | 10.58 | | 58.96 | 5.51 | 9.26 | 10.51 | |
| 7c | 60.34 | 5.66 | 8.80 | 10.08 | | 60.08 | 5.77 | 8.83 | 10.00 | |
| 7d | 54.38 | 3.85 | 10.63 | 12.34 | | 54.49 | 3.88 | 10.63 | 12.10 | |
| 7e | 55.60 | 4.49 | 9.98 | 11.42 | | 55.88 | 4.53 | 10.03 | 11.70 | |
| 7f | 62.56 | 4.32 | 8.53 | 9.82 | | 62.58 | 4.44 | 8.42 | 9.83 | |
| 7 g | 57.48 | 5.39 | 8.38 | 9.58 | | 57.29 | 5.11 | 8.31 | 9.55 | |
| 8a | 64.59 | 6.20 | 10.76 | 12.31 | | 64.40 | 6.11 | 10.68 | 12.15 | |
| 8b | 64.60 | 6.69 | 10.04 | 11.50 | | 64.53 | 6.59 | 10.38 | 11.41 | |
| 8c | 66.62 | 6.94 | 9.72 | 11.12 | | 66.37 | 6.77 | 9.65 | 10.81 | |
| 8e | 62.27 | 5.79 | 11.18 | 12.77 | | 62.63 | 5.55 | 11.03 | 12.52 | |
| 8f | 67.86 | 5.53 | 9.31 | 10.66 | | 67.86 | 5.50 | 9.24 | 10.61 | |
| 8 g | 63.13 | 6.62 | 9.20 | 10.53 | | 63.31 | 6.68 | 9.09 | 10.46 | |
| 9a | 48.93 | 4.19 | 19.02 | 10.89 | | 49.17 | 4.45 | 18.72 | 10.78 | |
| 9b | 44.89 | 3.43 | 19.04 | 10.90 | | 45.06 | 3.52 | 18.89 | 10.62 | |
| 9c | 50.94 | 3.80 | 17.60 | | | 50.99 | 3.99 | 17.83 | | |
| 9d | 51.53 | 3.71 | 12.88 | 9.83 | | 51.31 | 3.91 | 13.17 | 9.86 | |
| 9e | 48.89 | 2.84 | 13.16 | | | 48.64 | 3.06 | 12.88 | | |
| 9f | 40.34 | 3.74 | 14.86 | | | 40.63 | 3.43 | 15.16 | | |
| 9g | 45.11 | 3.79 | 21.04 | 12.04 | | 45.36 | 4.02 | 20.89 | 11.87 | |
| 9h | 50.18 | 3.45 | 15.96 | 12.18 | | 50.01 | 3.74 | 15.68 | 11.94 | |
| 10 | 53.60 | 4.46 | 14.43 | | | 53.82 | 4.19 | 14.18 | | |
| 11a | 62.46 | 5.55 | 9.71 | 11.12 | | 62.19 | 5.49 | 9.62 | 10.91 | |
| 11b | 59.99 | 6.24 | 8.74 | 9.99 | | 59.65 | 6.05 | 8.62 | 9.87 | |
| 11c | 60.27 | 6.65 | 8.27 | 9.45 | | 60.56 | 6.55 | 8.23 | 9.25 | |
| 11d | 67.71 | 7.58 | 7.52 | 8.60 | | 67.57 | 7.68 | 7.27 | 8.46 | |
| 11e | 69.87 | 5.86 | 7.40 | 8.44 | | 70.04 | 5.77 | 7.38 | 8.59 | |
| 11f | 61.45 | 6.02 | 8.43 | 9.64 | | 61.35 | 6.06 | 8.13 | 9.51 | |
| 11g | 63.86 | 6.82 | 8.27 | 9.47 | | 63.69 | 6.58 | 8.28 | 9.60 | |
| 12 | 61.12 | 9.62 | 8.91 | 20.35 | | 61.12 | 9.46 | 8.75 | 20.75 | |
| 13 | 58.66 | 6.71 | 6.22 | | | 58.47 | 6.51 | 6.36 | | |
| 14 | 54.21 | 5.75 | 5.75 | | 14.58 | 54.46 | 5.67 | 5.57 | | 14.77 |
| 15 | 66.09 | 6.67 | 4.06 | 9.27 | | 65.81 | 6.63 | 3.93 | 9.24 | |
| 16 | 69.19 | 7.03 | 5.04 | 11.53 | | 69.58 | 7.19 | 5.05 | 11.41 | |

**TABLEAU 2**

| **Activité anti-VIH-1 des composés selon l'invention** | | | |
|---|---|---|---|
| Composé | IC₅₀(nM) | CC₅₀(nM) | SI |
| AZT | 3 | >100000 | >33333 |
| 7a | 120 | >10000 | > 83 |
| 7b | =90 | > 10000 | >111 |
| 7c | =6 | >10000 | >1666 |
| 7d | >10000 | >10000 | - |
| 7e | 2800 | >10000 | >3 |
| 7f | 3800 | >100000 | >26 |
| 7g | 5 | >10000 | >2000 |
| 8a | 10 | >10000 | >1000 |
| 8b | 10 | >10000 | >1000 |
| 8c | 14 | >10000 | >714 |
| 8e | 1400 | 78000 | >55 |
| 8f | 250 | >9000 | >36 |
| 8g | 20 | >10000 | >500 |
| 9a | 17500 | 19500 | >1 |
| 9b | >100000 | >100000 | - |
| 9c | 43500 | 54500 | >1 |
| 9d | 5500 | 16000 | >2 |
| 9e | 14500 | 13500 | - |
| 9f | 17000 | 21500 | >1 |
| 9g | 56000 | 68500 | >1 |
| 9h | 18000 | 28500 | >1 |
| 10 | 8000 | 7500 | - |
| 11a | 6600 | >100000 | >15 |
| 11b | 67 | >100000 | >1492 |
| 11c | 41400 | >100000 | >2 |
| 11d- | >10000 | >1000 | - |
| 11e | >10000 | >10000 | - |
| 11f | 7500 | >100000 | >13 |
| 11g | 50 | >80000 | > 1600 |
| 15 | 3 | >10000 | > 3333 |
| 16 | 500 | >10000 | >20 |

**TABLEAU 3**

| **Activité anti-VIH-1 des composés selon l'invention à l'encontre d'une souche résistante à la Névirapine** | | | |
|---|---|---|---|
| Composé | IC₅₀(nM) | CC₅₀(nM) | SI |
| TIBO R32913* | >10000 | >10000 | - |
| 7a | >10000 | >10000 | - |
| 7b | 6700 | >10000 | >1 |
| 7c | 260 | >10000 | >38 |
| 7f | >10000 | >10000 | - |
| 8a | > 10000 | >10000 | - |
| 8b | 6700 | >10000 | >1 |
| 8c | 2100 | >10000 | >4 |
| 8e | 41500 | 78000 | >1 |
| 8f | 6600 | 8200 | >1.5 |
| 11b | >100000 | >100000 | - |
| 11g | 4300 | >100000 | >23 |
| 15 | 2200 | >10000 | >4.5 |

| | | | |
|---|---|---|---|
| * TIBO R 82913: 4,5,6,7-tétrahydro-5-méthyl-imidazo[4,5,1-jk][1,4-]-benzodiazepin-2(1H)-one. | | | |

**TABLEAU 4**

| **Activité anti-VIH-1 et VIH-2 du composé 7c** | | |
|---|---|---|
| | IC₅₀(M) | CC₅₀(M) |
| HIV-1 (LAI)/CEMSS | < 4 x 10⁻¹⁰ | > 10⁻⁵ |
| AZT (LAI)/CEMSS | 8.4 x 10⁻¹⁰ | |
| HIV-1 névirapine résistant/CEMSS | 2,3 x 10⁻⁸ | > 10⁻⁵ |
| HIV-1 IIIB/PBMC | < 3 x 10⁻¹¹ | > 10⁻⁵ |
| HIV-2 D194/PBMC | > 10⁻⁵ | > 10⁻⁵ |
| HTV-1 IIIB/MT4 | <10⁻¹² | > 10⁻⁵ |
| HIV-1 4200 type E(isolat primaire)/PBMC | < 10⁻¹⁰ | > 10⁻⁵ |
| HIV-1 6 type D(isolat primaire)/PBMC | < 10⁻¹⁰ | > 10⁻⁵ |

**TABLEAU 5**

| **Inhibition de la transcriptase inverse du VIH-** | |
|---|---|
| Composé | IC₅₀(nM) |
| HEPT * | >6000 |
| BPT ** | 400 |
| 7a | >10000 |
| 7c | 30 |
| 7e | >10000 |
| 8a | 100 |
| 8c | 15 |
| 8e | >4000 |
| 11b | >4000 |
| 11c | >4000 |
| 15 | 600 |
| 16 | 400 |

| | |
|---|---|
| * HEPT = 1-[(2-hydroxyéthoxyméthyl]-6-(phénylthio)-thymine | |
| ** BPT = 1-benzyloxyméthyl-6--phénylthio)thymine. | |

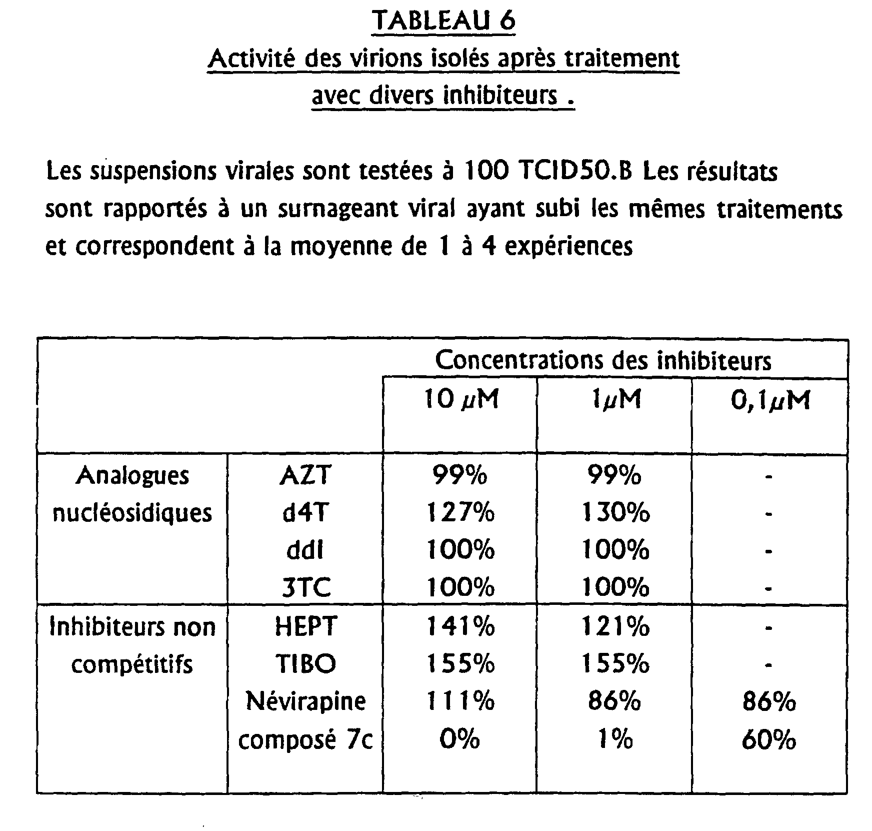

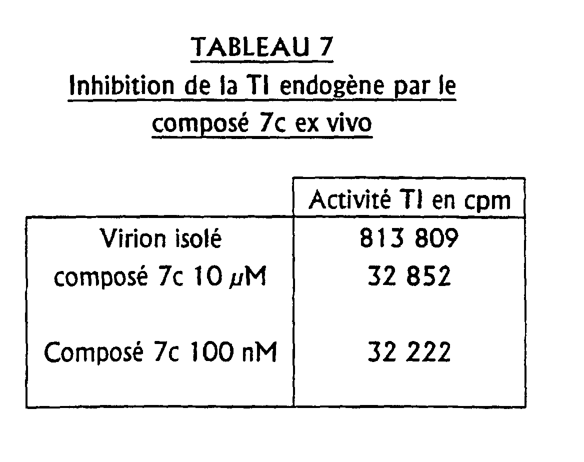

## Revendications

1. Composés de formule (3): 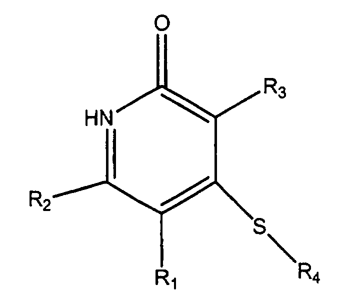 dans laquelle:
- R₁ et R₂ représentent indépendamment un atome d'hydrogène, un groupe aliphatique ou un groupe alkyloxyalkyle dans lequel les chaînes alkyle sont en C₁ à C₄, ou forment ensemble un cycle aromatique;
- R₃ représente NN₂, NHCOCH₃, NO₂ ou COOC₂H₅;
- R₄ représente un groupe phényle, pyrimidine, benzimidazole, benzoxazole, benzothiazole, thiazoline, imidazole ou pyridine, éventuellement substitué par un ou plusieurs groupes aliphatiques et/ou par un ou plusieurs groupes hydroxy
à l'exclusion du composé dans lequel R₁ représente un atome d'hydrogène, R₂ représente CH₃, R₃ représente CO₂C₂H₅ et R₄ représente un groupe phényle.

2. Composé selon la revendication 1 **caractérisé en ce que** R₁ et R₂ représentent indépendamment un atome d'hydrogène ou un groupe alkyle en C₁ à C₄ ou un groupe alkyloxyméthyle.

3. Composés selon l'une des revendications 1 à 2 **caractérisés en ce que** R₄ est un groupe phényle substitué par deux groupes méthyle.

4. Composés selon la revendication 3, **caractérisés en ce que** les deux groupes méthyle sont positionnés en méta du groupe phényle.

5. Composé selon l'une des revendications 1 à 4 **caractérisé en ce qu'**il est la 5-éthyl-6-méthyl-3-carbéthoxy-4-(3',5'-diméthylphényl)thio-pyridin-2(1H)one.

6. Composé selon l'une des revendications 1 à 4, **caractérisé en ce qu'**il est la 5-éthyl-6-méthyl-3-nitro-4-(3',5'-diméthylphényl)thio-pyridin-2(1H)-one.

7. Composé selon l'une des revendications 1 à 4, **caractérisé en ce qu'**il est la 3-amino-5-éthyl-6-méthyl-4-(3',5'-diméthylphényl)thio-pyridin-2(1H)-one.

8. Composé selon l'une des revendications 1 à 4 **caractérisé en ce qu'**il est la 3-amino-5-méthyl-4-(3',5'-diméthylphényl)thio-pyridin-2(1H)-one.

9. Composé selon l'une des revendications 1 à 4, **caractérisé en ce qu'**il est la 3-amino-5-éthyl-4-(3',5'-diméthylphényl)thio-pyridin-2(1H)-one.

10. Composé selon l'une quelconque des revendications 1 à 4 **caractérisé en ce qu'**il est la 3-amino-5-éthoxyméthyl-4-(3',5'-diméthylphényl)thio-pyridin-2(1H)-one (formule 8g).

11. Composé selon l'une des revendications 1 à 4 **caractérisé en ce que** la 5-éthoxyméthyl-3-nitro-4-(3',5'-diméthylphényl)thio-pyridin-2(1H)-one (formule 7g).

12. Procédé de fabrication de composés selon l'une des revendications 1 à 4 dans lesquels R₃ représente NO₂, **caractérisé en ce que** l'on fait réagir une chloronitropyridinone de formule (6) suivante: 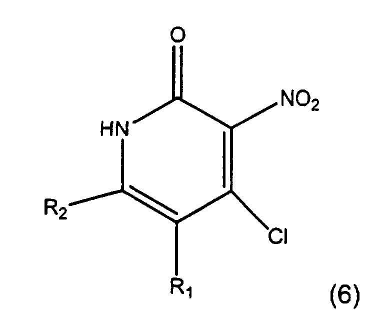 avec un thio phénol, ou un dérivé mercapto d'un hétérocycle, éventuellement substitué de formule R₄SH.

13. Procédé de fabrication de composés selon l'une des revendications 1 à 4 dans lequel R₃ représente NH₂ **caractérisé en ce que** l'on réduit en présence de chlorure stanneux hydraté ou par hydrogénation catalytique une nitropyridinone obtenue selon la revendication 12.

14. Procédé de fabrication d'un composé selon la revendication 1, dans lequel R₃ représente NHCOCH₃ et R₄ représente un groupement phényle éventuellement substitué, **caractérisé en ce que** l'on fait réagir un composé obtenu selon la revendication 13 en présence d'un composé de formule (CH₃-CO)₂O ou CH₃COZ, dans lequel Z est un radical susceptible de se libérer et de permettre la formation d'une liaison amide.

15. Procédé de fabrication d'un composé selon la revendication 1 dans lequel R₃ représente un groupe COOC₂H₅ et R₄ représente un groupe phényle éventuellement substitué, **caractérisé en ce que** l'on fait réagir une 4-chloropyridinone de formule (14) suivante : 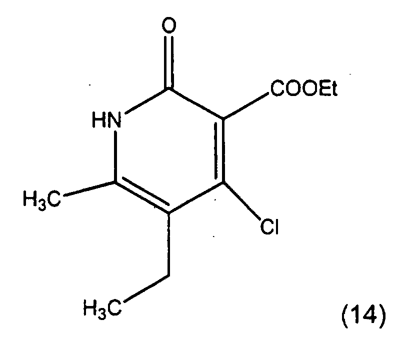 avec un thio phénol éventuellement substitué.

16. Procédé selon la revendication 15 **caractérisé en ce que** la 4-chloropyridinone est obtenue par réaction de l'hydroxypyridinone de formule (13) suivante : 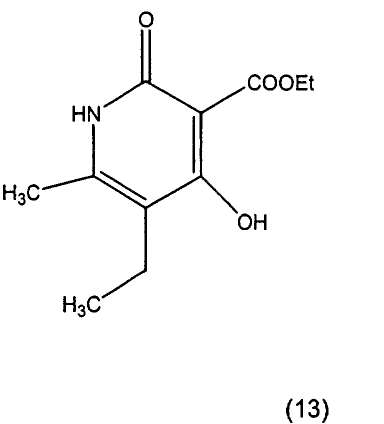 en présence de POCI₃.

17. Procédé selon la revendication 12, **caractérisé en ce que** la chloronitropyridinone de formule (6) est obtenue par traitement de la nitropyridinone correspondante par POCl₃.

18. Composé **caractérisé en ce qu'**il présente la formule (6) suivante, dans laquelle R₁ et R₂ représentent indépendamment des groupes éthyle ou méthyle.

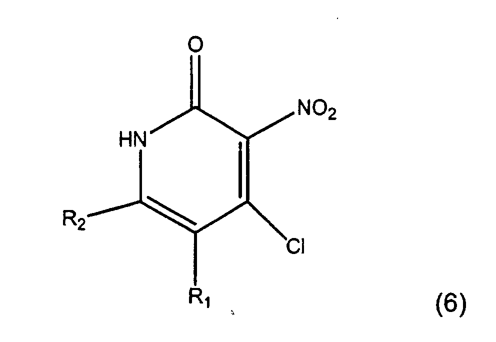

19. Composé **caractérisé en ce qu'**il présente la formule (17) suivante, dans laquelle R₁ et R₂ représentent indépendamment des groupes éthyle ou méthyle

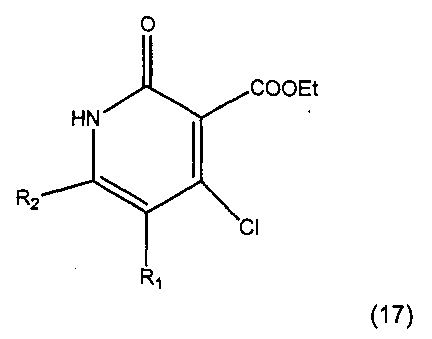

20. Composition pharmaceutique **caractérisée en ce qu'**elle contient une quantité efficace d'un composé selon l'une des revendications 1 à 11 ou obtenu par un procédé selon l'une des revendications 12 à 17 en mélange avec des excipients compatibles.

21. Médicament **caractérisé en ce qu'**il contient un composé selon l'une des revendications 1 à 11 ou obtenu par un procédé selon l'une des revendications 12 à 17.

22. Utilisation d'un composé selon l'une des revendications 1 à 11 ou obtenu par un procédé selon l'une des revendications 12 à 17 pour la fabrication d'un médicament pour le traitement des maladies liées aux VIH.

## Patentansprüche

1. Verbindungen der Formel (3): 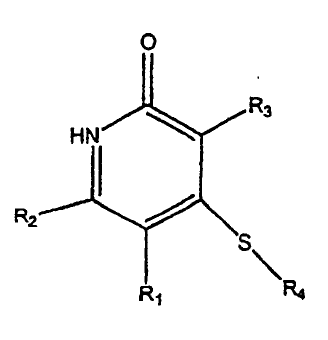 wobei:
- R₁ und R₂ unabhängig voneinander ein Wasserstoffatom, eine aliphatische Gruppe oder eine Alkyloxyalkylgruppe, in der die Alkylketten C₁- bis C₄-Gruppen sind, darstellen oder zusammen einen aromatischen Ring bilden;
- R₃ NH₂, NHCOCH₃, NO₂ oder COOC₂H₅ darstellt;
- R₄ eine Phenyl-, Pyrimidin-, Benzimidazol-, Benzoxazol-, Benzothiazol-, Thiazolin-, Imidazol- oder Pyridingruppe darstellt, die gegebenenfalls mit einer oder mehreren aliphatischen Gruppen und/oder mit einer oder mehreren Hydroxygruppen substituiert ist;
wobei die Verbindung ausgeschlossen ist, bei der R₁ ein Wasserstoffatom darstellt, R₂ CH₃ darstellt, Rₐ CO₂C₂H₅ darstellt und R₄ eine Phenylgruppe darstellt.

2. Verbindung gemäß Anspruch 1, **dadurch gekennzeichnet, dass** R₁ und R₂ unabhängig voneinander ein Wasserstoffatom oder eine C₁- bis C₄-Alkylgruppe oder eine Alkyloxymethylgruppe darstellen.

3. Verbindungen gemäß einem der Ansprüche 1 bis 2, **dadurch gekennzeichnet, dass** R₄ eine Phenylgruppe ist, die mit zwei Methylgruppen substituiert ist.

4. Verbindungen gemäß Anspruch 3, **dadurch gekennzeichnet, dass** sich die zwei Methylgruppen in meta-Stellung der Phenylgruppe befinden.

5. Verbindung gemäß einem der Ansprüche 1 bis 4, **dadurch gekennzeichnet, dass** es sich um 5-Ethyl-6-methyl-3-carbethoxy-4-(3',5'-dimethylphenyl)-thiopyridin-2(1H)-on handelt.

6. Verbindung gemäß einem der Ansprüche 1 bis 4, **dadurch gekennzeichnet, dass** es sich um 5-Ethyl-6-methyl-3-nitro-4-(3',5'-dimethylphenyl)thiopyridin-2(1H)-on handelt.

7. Verbindung gemäß einem der Ansprüche 1 bis 4, **dadurch gekennzeichnet, dass** es sich um 3-Amino-5-ethyl-6-methyl-4-(3',5'-dimethylphenyl)thiopyridin-2(1H)-on handelt.

8. Verbindung gemäß einem der Ansprüche 1 bis 4, **dadurch gekennzeichnet, dass** es sich um 3-Amino-5-methyl-4-(3',5'-dimethylphenyl)thiopyridin-2(1H)-on handelt.

9. Verbindung gemäß einem der Ansprüche 1 bis 4, **dadurch gekennzeichnet, dass** es sich um 3-Amino-5-ethyl-4-(3',5'-dimethylphenyl)thiopyridin-2(1H)-on handelt.

10. Verbindung gemäß einem der Ansprüche 1 bis 4, **dadurch gekennzeichnet, dass** es sich um 3-Amino-5-ethoxymethyl-4-(3',5'-dimethylphenyl)thiopyridin-2(1H)-on (Formel 8g) handelt.

11. Verbindung gemäß einem der Ansprüche 1 bis 4, **dadurch gekennzeichnet, dass** es sich um 5-Ethoxymethyl-3-nitro-4-(3',5'-dimethylphenyl)thiopyridin-2(1H)-on (Formel 7g) handelt.

12. Verfahren zur Herstellung von Verbindungen gemäß einem der Ansprüche 1 bis 4, wobei R₃ NO₂ darstellt, **dadurch gekennzeichnet, dass** man ein Chlornitropyridon der folgenden Formel (6): 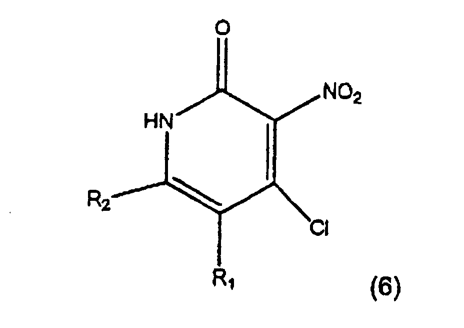 mit einem gegebenenfalls substituierten Thiophenol oder Mercaptoderivat eines Heterocyclus der Formel R₄SH umsetzt.

13. Verfahren zur Herstellung von Verbindungen gemäß einem der Ansprüche 1 bis 4, wobei R₃ NH₂ darstellt, **dadurch gekennzeichnet, dass** man ein gemäß Anspruch 12 erhaltenes Nitropyridinon in Gegenwart von Zinn(II)-chlorid-Hydrat oder durch katalytische Hydrierung reduziert.

14. Verfahren zur Herstellung einer Verbindung gemäß Anspruch 1, wobei R₃ NHCOCH₃ darstellt und R₄ eine gegebenenfalls substituierte Phenylgruppe darstellt, **dadurch gekennzeichnet, dass** man eine gemäß Anspruch 13 erhaltene Verbindung in Gegenwart einer Verbindung der Formel (CH₃-CO)₂O oder CH₃COZ umsetzt, wobei Z ein Rest ist, der freigesetzt werden und die Bildung einer Amidbindung ermöglichen kann.

15. Verfahren zur Herstellung einer Verbindung gemäß Anspruch 1, wobei R₃ eine COOC₂H₅-Gruppe darstellt und R₄ eine gegebenenfalls substituierte Phenylgruppe darstellt, **dadurch gekennzeichnet, dass** man ein 4-Chlorpyridinon der folgenden Formel (14): 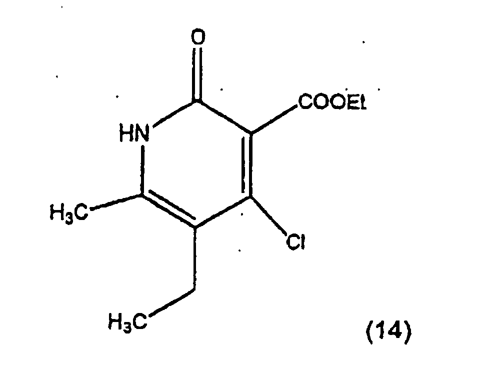 mit einem gegebenenfalls substituierten Thiophenol umsetzt.

16. Verfahren gemäß Anspruch 15, **dadurch gekennzeichnet, dass** das 4-Chlorpyridinon durch Reaktion von Hydroxypyridinon der folgenden Formel (13): 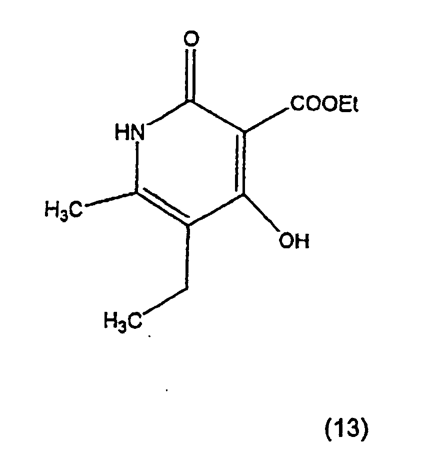 in Gegenwart von POCl₃ erhalten wird.

17. Verfahren gemäß Anspruch 12, **dadurch gekennzeichnet, dass** das Chlornitropyridinon der Formel (6) durch Behandlung des entsprechenden Nitropyridinons mit POCI₃ erhalten wird.

18. Verbindung, **dadurch gekennzeichnet, dass** sie die folgende Formel (6) darstellt, wobei R₁ und R₂ unabhängig voneinander Ethyl- oder Methylgruppen darstellen:

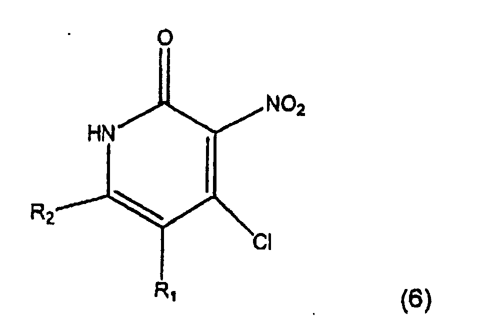

19. Verbindung, **dadurch gekennzeichnet, dass** sie die folgende Formel (17) darstellt, wobei R₁ und R₂ unabhängig voneinander Ethyl- oder Methylgruppen darstellen:

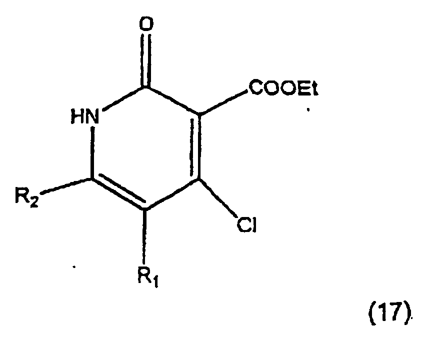

20. Pharmazeutische Zusammensetzung, **dadurch gekennzeichnet, dass** sie eine wirksame Menge einer Verbindung gemäß einem der Ansprüche 1 bis 11 oder einer Verbindung, die durch ein Verfahren gemäß einem der Ansprüche 12 bis 17 erhalten wurde, im Gemisch mit verträglichen Trägerstoffen enthält.

21. Medikament, **dadurch gekennzeichnet, dass** es eine Verbindung gemäß einem der Ansprüche 1 bis 11 oder eine Verbindung, die durch ein Verfahren gemäß einem der Ansprüche 12 bis 17 erhalten wurde, enthält.

22. Verwendung einer Verbindung gemäß einem der Ansprüche 1 bis 11 oder einer Verbindung, die durch ein Verfahren gemäß einem der Ansprüche 12 bis 17 erhalten wurde, zur Herstellung eines Medikaments zur Behandlung von Krankheiten, die mit HIV verbunden sind.

## Claims

1. Compounds of formula (3) : 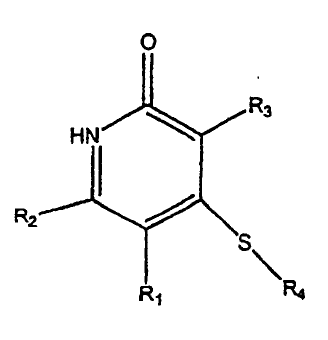 wherein
- R₁ and R₂ independently represent an hydrogen, an aliphatic group or an alkyloxyalkyl group in which the alkyl groups are C₁ to C₄ groups or together form an aromatic ring;
- R₃ represents : NH₂, NHCOCH₃, NO₂ or COOC₂H₅;
- R₄ represents a phenyl, pyrimidine, benzimidazole, benzoxazole, benzothiazole, thiazoline, imidazole or pyridine group optionally substituted with one ore more aliphatic groups and/or with one or more hydroxyl groups;
with the exclusion of the compound in which R₁ represents an hydrogen, R₂ represents CH₃, R₃ represents CO₂C₂H₅ and R₄ represents a phenyl group.

2. Compound according to Claim 1, **characterised in that** R₁ and R₂ independently represent an hydrogen or a C₁ to C₄ alkyl group or an alkyloxymethyl group.

3. Compounds according to one of Claims 1 and 2, **characterised in that** R₄ is a phenyl group substituted with two methyl groups.

4. Compounds according to Claim 3, **characterised in that** the two methyl groups are located in the meta position of the phenyl group.

5. Compound according to one of Claims 1 to 4, **characterised in that** it is 5-ethyl-6-methyl-3-carbethoxy-4-(3',5'-dimethylphenyl)thio-pyridin-2(1H)-one.

6. Compound according to one of Claims 1 to 4, **characterised in that** it is 5-ethyl-6-methyl-3-nitro-4-(3',5'-dimethylphenyl)thio-pyridin-2(1H)-one.

7. Compound according to one of Claims 1 to 4, **characterised in that** it is 3-amino-5-ethyl-6-methyl-4-(3',5'-dimethylphenyl)thio-pyridin-2(1H)-one.

8. Compound according to one of Claims 1 to 4, **characterised in that** it is 3-amino-5-methyl-4-(3',5'-dimethylphenyl)thio-pyridin-2(1H)-one.

9. Compound according to one of Claims 1 to 4, **characterised in that** it is 3-amino-5-ethyl-4-(3',5'-dimethylphenyl)thio-pyridin-2(1H)-one.

10. Compound according to any one of Claims 1 to 4, **characterised in that** it is 3-amino-5-ethoxymethyl-4-(3',5'-dimethylphenyl)thio-pyridin-2(1H)-one (formula 8g).

11. Compound according to one of Claims 1 to 4, **characterised in that** it is 5-ethoxylmethyl-3-nitro-4-(3',5'-dimethylphenyl)thio-pyridin-2(1H)-one (formula 7g).

12. A method of producing compounds according to one of Claims 1 to 4, in which R₃ represents NO_{2,} **characterised in that** the compounds are formed by reacting a chloronitropyridinone of following formula (6) : 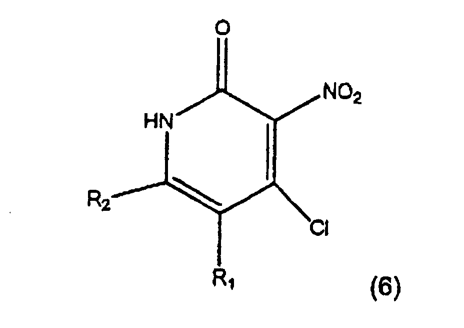 with a thio-phenol or a mercapto derivative of an optionally substituted heterocyclic compound of formula R₄SH.

13. A method of producing compounds according to one of Claims 1 to 4, in which R₃ represents NH₂, **characterised in that** the compounds are formed by reducing a nitropyridinone obtained according to Claim 12, in the presence of hydrated stannous chloride or by catalytic hydrogenation.

14. A method of producing a compound according to Claim 1, in which R₃ represents NHCOCH₃ and R₄ represents an optionally substituted phenyl group **characterised in that** the compound is formed by reacting a compound obtained according to Claim 13 in the presence of a compound of formula (CH₃-CO)₂O or CH₃COZ, in which Z is a leaving liable radical and liable to allow the formation of an amide bond.

15. A method of producing a compound according to Claim 1, in which R₃ represents COOC₂H₅ group and R₄ represents an optionally substituted phenyl group
**characterised in that** the compound is formed by reacting a 4-chloropyridinone of following formula (14) : 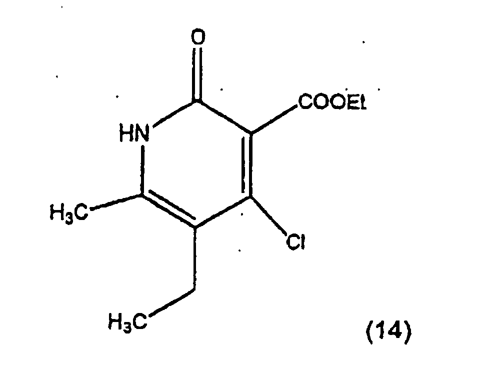 with an optionally substituted thio-phenol.

16. A method according to Claim 15, **characterised in that** the 4-chloropyridinone is obtained by reaction of the hydroxypyridinone of following formula (13) : 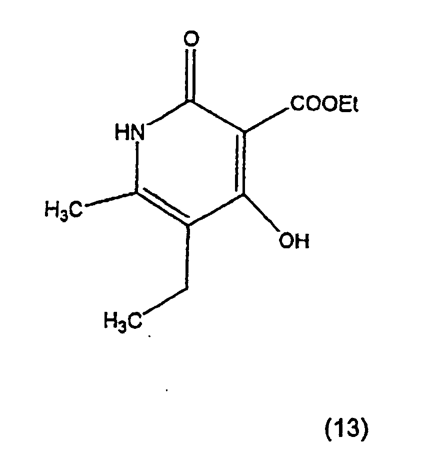 in the presence of POCI₃.

17. A method according to Claim 12, **characterised in that** the chloronitropyridinone of formula (6) is obtained by treatment of the corresponding nitropyridinone with POCl₃.

18. A compound **characterised in that** it has the following formula (6), in which R₁ and R₂ independently represent ethyl or methyl groups.

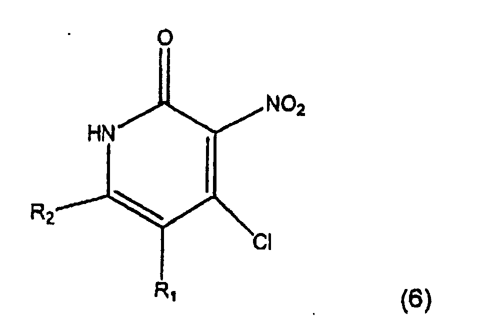

19. A compound **characterised in that** it has the following formula (17), in which R₁ and R₂ independently represent ethyl or methyl groups.

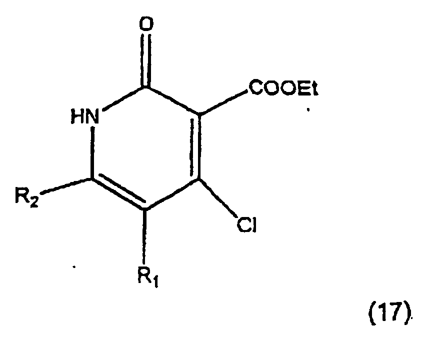

20. A pharmaceutical composition **characterised in that** it comprises an effective quantity of a compound according to one of Claims 1 to 11 or obtained by a method according to one of Claims 12 to 17 with suitable vehicles.

21. A drug **characterised in that** it comprises a compound according to one of Claims 1 to 11 or obtained by a method according to one of Claims 12 to 17.

22. Use of a compound according to one of Claims 1 to 11 or obtained by a method according to the one of Claims 12 to 17 for the production of a drug for the treatment of illnesses linked to HIV.
